# EUROPEAN PATENT SPECIFICATION

(11) **EP 3 820 350 B1**
(45) Date of publication and mention of the grant of the patent: **25.03.2026**
(21) Application number: 19745916.7
(22) Date of filing: 10.07.2019
(51) Int. Cl.: A61B 1/00, A61B 50/30

(54) **TIP PROTECTOR**
SPITZENSCHUTZ
PROTECTEUR DE POINTE

(30) Priority: 10.07.2018 US 201862696244 P
(43) Date of publication of application: 19.05.2021
(73) Proprietor: United States Endoscopy Group, Inc., Mentor, OH 44060 (US)
(72) Inventor: KAYE, Christopher J., Middleburg Heights, Ohio 44130 (US); MANN, Gary E., Painesville, Ohio 44077 (US); MOORE, Craig, Pepper Pike, Ohio 44124 (US)
(74) Representative: Petraz, Gilberto Luigi
(86) International application number: PCT/US2019/041243
(87) International publication number: WO 2020/014390

(56) References cited:
- JP-A- 2007 075 281
- JP-U- H0 515 901
- US-A1- 2014 343 361
- US-A1- 2015 069 728

## Description

### BACKGROUND

Endoscopes are well-known in the art and are commonly used for numerous medical procedures. Endoscopes often include fragile components that can be easily damaged or broken, such as, for example, a camera lens, a light source, drive wires and connections for the camera lens and light source, or the like. During endoscopic medical procedures, the camera and light source for these devices are used to capture images (e.g., images of a patient's digestive tract). Document JP H05 15901 U discloses an endoscope tip protector comprising a cylindrical cage and a plurality of elastic flaps extending inwardly to removably attach the distal tip of an endoscope.

### SUMMARY

Tip protectors are often used to protect the fragile components in the distal tip of an endoscope while the endoscope is being stored. An exemplary tip protector for attaching to a distal tip of an endoscope is defined in annexed independent claim 1. Other advantageous features are defined in the annexed dependent claims.

### BRIEF DESCRIPTION OF THE DRAWINGS

FIG. 1A is a cross-sectional view of an exemplary embodiment of a tip protector attached to a distal tip of an endoscope;
FIG. 1B is a cross sectional view of the exemplary embodiment of the tip protector of FIG 1A taken along the line 1B-1B of FIG. 1A;
FIG. 2A is a cross-sectional view of another exemplary embodiment of a tip protector attached to a distal tip of an endoscope;
FIG. 2B is a cross sectional view of the exemplary embodiment of the tip protector of FIG 14A taken along the line 2B-2B of FIG. 2A;
FIG. 3A is a perspective view of another exemplary embodiment of a tip protector;
FIG. 3B is side view of the exemplary embodiment of the tip protector of FIG. 3A;
FIG. 3C is a front view of the exemplary embodiment of the tip protector of FIG. 3A;
FIG. 3D is perspective view of an exemplary embodiment of a web member of an exemplary embodiment of a positional membrane for the tip protector of FIG. 3A;
FIG. 3E is a side view of the exemplary embodiment of the web member shown in FIG. 3D;
FIG. 4A is a partial cross-sectional view of the exemplary tip protector of FIGS. 3A-3E taken along the line 4A-4A in FIG. 3B;
FIG. 4B is a partial side view of the exemplary tip protector of FIG. 3A;
FIG. 5A is a perspective view of another exemplary embodiment of a tip protector;
FIG. 5B is side view of the exemplary embodiment of the tip protector of FIG. 5A;
FIG. 5C is a front view of the exemplary embodiment of the tip protector of FIG. 5A;
FIG. 6A is a perspective view of another exemplary embodiment of a tip protector;
FIG. 6B is side view of the exemplary embodiment of the tip protector of FIG. 6A;
FIG. 6C is a front view of the exemplary embodiment of the tip protector of FIG. 6A;
FIG. 7 illustrates another exemplary embodiment of a tip protector;
FIG. 8A illustrates another exemplary embodiment of a tip protector, in which the tip protector is attached to a distal tip of an endoscope;
FIG. 8B illustrates a proximal end of the tip protector of FIG. 8A;
FIG. 8C illustrates a distal end of the tip protector of FIG. 8A;
FIG. 9 illustrates an exemplary embodiment of a positional membrane for a tip protector;
FIG. 10 illustrates another exemplary embodiment of a tip protector having a hinge that allows the tip protector to be moved between an open position and a closed position, in which the tip protector is shown in the open position;
FIG. 11A illustrates another exemplary embodiment of a tip protector having a hinge that allows the tip protector between an open position and a closed position, in which the tip protector is shown in the open position;
FIG. 11B illustrates the exemplary embodiment of the tip protector of FIG. 11A, in which the tip protector is shown in the closed position;
FIG. 12A illustrates another exemplary embodiment of a tip protector having multiple hinges that allows the tip protector between an open position and a closed position, in which the tip protector is shown in the open position; and
FIG. 12B illustrates the exemplary embodiment of the tip protector of FIG. 12A, in which the tip protector is shown in the closed position;
FIG. 13A is a perspective view of an exemplary embodiment of a free-standing positional membrane for a tip protector, in which the free-standing positional membrane is attached to an endoscope;
FIG. 13B is perspective view of an exemplary embodiment of a cage for a tip protector engaging the free-standing positional membrane of FIG. 13A, in which the cage is maintained in a position to protect a distal tip of the endoscope by the engagement between the exemplary free-standing positional membrane of FIG. 13A;
FIG. 14A is a side view of another exemplary embodiment of a tip protector;
FIG. 14B is a front view of the exemplary embodiment of the tip protector of FIG. 14A;
FIG. 14C is a cross-sectional view of the tip protector of FIG. 14B taken along the lines
B-B of FIG 14B;
FIG. 14D is a detail view of the tip protector of FIG. 14C taken along the line C of FIG. 14C;
FIG. 15 illustrates a duodenoscope, in which an elevator of the duodenoscope is extending from the distal tip of the duodenoscope at an angle;
FIG. 16 illustrates an exemplary embodiment of a cap for the elevator of a duodenoscope;
FIG. 17A illustrates an exemplary embodiment of a protective sleeve for protecting an endoscope during transportation and storage, in which the protective sleeve is attached along the entire length of the endoscope;
FIG. 17B illustrates another exemplary embodiment of a protective sleeve for protecting an endoscope during transportation and storage, in which the protective sleeve is attached to the distal end of the endoscope;
FIG. 17C illustrates another exemplary embodiment of a protective sleeve for protecting an endoscope during transportation and storage, in which the protective sleeve is attached to the proximal end of the endoscope;
FIG. 18 illustrates a further exemplary embodiment of a tip protector in accordance with the disclosure provided herein; and
FIG. 19 illustrates yet a further exemplary embodiment of a tip protector in accordance with the disclosure provided herein.

### DETAILED DESCRIPTION

The Detailed Description describes exemplary embodiments of the invention and is not intended to limit the scope of the claims in any way. Indeed, the invention is broader than and unlimited by the exemplary embodiments, and the terms used in the claims have their full ordinary meaning. Features and components of one exemplary embodiment may be incorporated into the other exemplary embodiments. Inventions within the scope of this application may include additional features, or may have less features, than those shown in the exemplary embodiments.

Endoscopes are optical, tubular instruments that are used in various medical procedures to capture images within a patient's body. Endoscopes often include fragile components (e.g., a camera lens, a light source, drive wires and connections for the camera lens and light source, or various other delicate items) that are critical for endoscopic procedures. These components are located at a distal tip of the endoscope. Not only can these components be fragile, but they are often the most expensive part of the endoscope. Accordingly, it is advantageous to transport and store endoscopes in a manner that prevents these fragile and expensive components from being damaged. Accordingly, a tip protector is often used to protect the distal tip of an endoscope during storage and transportation.

In addition, because endoscopes are placed inside a patient's body, the endoscopes must be sterilized or receive high-level disinfection (HLD) prior to being used again. After each use, the endoscope is thoroughly cleaned, which can result in residual moisture remaining on the endoscope, especially within the accessory channel. If residual moisture is trapped in a scope location that is subject to minimal air flow, bacteria growth can occur and cause the endoscope to be compromised. It is advantageous to use a tip protector that minimizes or leads to increased elimination of residual moisture that is trapped, thereby decreasing the likelihood of bacteria growth on the endoscope.

Tip protectors are connected to an endoscope such that the tip protector surrounds the scope's distal tip. In addition to the air flow features, the tip protector may also prevent outside objects from impacting the distal tip during transportation and storage of the endoscope. Some tip protectors contact the endoscope at multiple locations, including contacting the scope's distal tip. These contact points between the tip protector and the endoscope often provide areas where residual moisture can become trapped, which can lead to bacteria. Some tip protectors are made of foam, and these foam-type tip protectors may trap moisture. Therefore, it is advantageous to use a tip protector that reduces the number of contact points between the tip protector and the endoscope. It is also advantageous to use a tip protector that prevents residual moisture from being trapped at these contact points. In addition, it is advantageous to use a tip protector that does not trap contaminants in the endoscope. It is further advantageous to use a tip protector that indicates if the tip protector has contacted moisture and/or the endoscope are in a compromised state with respect to infection.

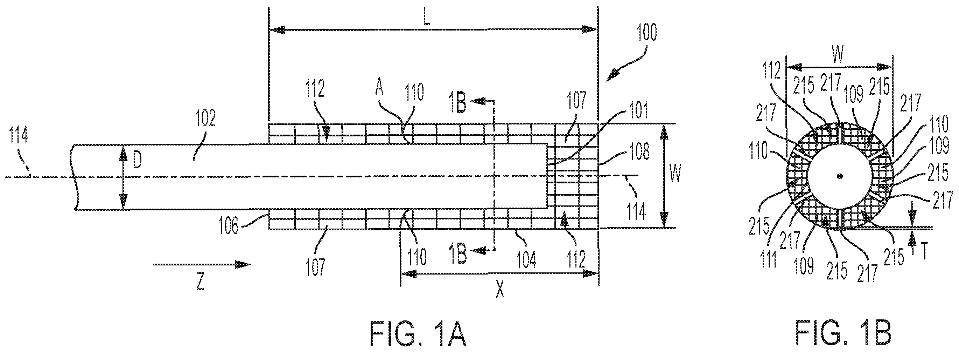

Referring to FIGS. 1A and 1B, an exemplary embodiment of a tip protector 100 is attached to a distal tip 101 of an endoscope 102 such that the tip protector is positioned to protect one or more of the endoscope's tip components during storage. The tip protector 100 includes a cage 104 and at least one positional membrane 110. The cage 104, when attached, has a proximal end 106, a distal end 108, and a plurality of holes 107 disposed between the proximal distal ends. The holes 107 allow for air flow to facilitate drying of any residual moisture on an attached endoscope, and provides a pathway for any residual moisture on an attached endoscope to exit. The cage 104 defines a channel 112 that extends along the length L of the cage 104 between the proximal end 106 and the distal end 108. In the illustrated embodiment, the cross-sectional shape of the channel 112 and the cage 104 is circular (as shown in FIG. 1B). In other embodiments, the cross-sectional shape of the channel 112 and the cage 104 can take any other suitable shape, such as, for example, polygonal, oval, or other suitable shape. In some embodiments, the proximal end 106 and/or the distal end 108 of the cage 104 can be open such that air flow can enter the channel 112 to facilitate drying of any residual moisture on an attached endoscope. In certain embodiments, the tip protector 100 is configured such that a center of the channel 112, a center of the proximal end 106, and a center of the distal end 108 are aligned on an axis 114.

When the tip protector 100 is attached to the endoscope 102, the distal tip 101 of the endoscope is disposed within the channel 112 of the cage 104, such that the cage 104 surrounds the distal tip 101. In certain embodiments, the cage 104 is made from a soft material such as, for example, thermoplastic elastomer (TPE), flexible PVC, Polyurethane, Silicone, other polymeric material, paper-based material, etc. The soft material prevents the distal tip 101 from being damaged due to contact with the cage 104. The length L of the cage 104 can be between about 1" and about 9". In certain embodiments, the width W between the inner surfaces of the cage 104 can be between about 0.125" and about 2.75" greater than the diameter D of the endoscope 102 it is meant to protect. In certain embodiments, the width W between the inner surfaces of the cage 104 can be between about 0.425" and about 3.25". A thickness T of the cage 104 can be between about 0.020" and about 0.150".

The above- mentioned ranges for the width W between the inner surfaces of the cage 104 and thickness T are exemplary. It should be understood that the cage 104 can be manufactured to have any suitable width W and thickness T based on the endoscope (or other medical device) that it is meant to protect.

In the illustrated embodiment, the tip protector 100 has one positional membrane 110, but it should be understood that the tip protector 100 could have more than one such membrane. The positional membrane 110 is connected, joined or integral to the cage 104 and configured to contact the endoscope 102 while securing the tip protector 100 to the endoscope. In certain embodiments, the positional membrane 110 is configured such that the only contact between the endoscope 102 and the tip protector 100 is between the positional membrane and the endoscope. That is, there is no contact between the cage 104 and the endoscope 102 during storage of the endoscope. In some situations, the endoscope 102 can move within the cage 104 to cause minimal contact between the cage and the endoscope (rather than no contact), but the tip protector 100 can be configured such that there is no contact between the cage 104 and the endoscope 102 when the endoscope is properly secured to the tip protector by the positional membrane 110. As described above, residual moisture on an endoscope 102 often resides at the connection between an endoscope and a tip protector. As a result, the tip protector 100 described herein is advantageous because it reduces the contact points between the endoscope 102 and the tip protector 100. In turn, this reduces the areas where residual moisture can reside.

In FIGS. 1A and 1B, the positional membrane 110 also includes a main opening 111 configured to receive the endoscope and a plurality of air flow openings 109 that allow for air flow through the positional membrane such that any residual moisture can dry or exit during storage of the endoscope. In certain embodiments, the main opening 111 of the positional membrane is aligned with the axis 114 that extends between the proximal end 106 and the distal end 108 of the cage 104.

The positional membrane 110 is flexible such that it is movable between a normal position (i.e., the position where the tip protector is not attached to an endoscope) and an attached position (i.e., the position where the tip protector is attached to an endoscope). In some embodiments, when the positional membrane 110 is in the normal position, the positional membrane is substantially planar, and, when the positional membrane 110 moves from the normal position to the attached position, the positional membrane 110 flexes such that the main opening 111 expands to receive an endoscope. Turning to Fig. 1B, the positional membrane 110 includes a plurality of web members 215 that surround the main opening 111. Each of the web members 215 are separated from each other by a slot 217 such that the web members can move independently of each other. The slots 217 also allow the main opening 111 to expand. The web members 215 include air flow openings 109 that allow for air flow through the positional membrane 110 such that residual moisture on an attached endoscope can dry and/or exit the tip protector 100 during storage.

Referring to FIG. 1A (which shows the positional membrane in the attached position), the positional membrane 110 is flexed toward the distal end 108 of the endoscope, which causes the main opening 111 to be in an expanded position. In particular, the main opening 111 expands to correspond to the diameter D of the endoscope 102. In the illustrated embodiment, the positional membrane 110 flexes towards the distal end 108 of the cage 104 because the distal tip 101 of the endoscope was inserted into the tip protector 101 in the direction Z through the proximal end 106 and toward the distal end 108.

While the flexibility of the positional membrane 110 allows the main opening 111 to expand as needed, the positional membrane is configured to remain or move back to the normal position when it is not in use. This desired movement back to the normal position causes the positional membrane 110 to place a force on the endoscope 102 resulting in entire tip protector being secured to the endoscope. Because the positional membrane 110 provides a force on the endoscope 102, movement of the endoscope (after being attached to the positional membrane

110) can cause the positional membrane 110 to bend or flex. In some instances, repositioning the endoscope 102 within the tip protector will result in the positional membrane 110 flexing toward the proximal end 106 of the endoscope, rather than the distal end 108 of the endoscope

102. The direction that the positional membrane 110 flexes (e.g., toward the proximal end 106 or the distal end 108) does not affect the ability of the positional membrane 110 to secure the tip protector 100 to the endoscope 102. That is, in either of the instances described above, the positional membrane 110 will tend to move back to its normal or original position. In turn, this will create a force that causes the tip protector to be secured to the endoscope.

The positional membrane 110 can be made of any suitable flexible material that is capable of securing the tip protector 100 to an endoscope. Thermoplastic elastomer (TPE), flexible PVC, Polyurethane, Silicone, or other polymeric material, or paper-based material, etc. are suitable examples. Moreover, material of the positional membrane 110 can be selected based on the characteristics of the endoscope 102 being protected. A harder material 110 (e.g., a less compliant material) can be used for an endoscope 102 having a smaller diameter, and a softer material (e.g., a more compliant material) can be used for an endoscope 102 having a larger diameter. It is advantageous to use a positional membrane 110 made of a hard material for a smaller diameter endoscope 102 as the hard material exerts more force on the endoscope (as compared to a soft material) to better secure the tip protector 100 to the endoscope. Likewise, a positional membrane made of a soft material will flex more (as compared to a hard material) such that the main opening 111 is larger for receiving the larger diameter endoscope.

In certain embodiments, a thickness R of the positional membrane 110 can be between about 0.015" and about 0.125". In other embodiments, the thickness R of the positional membrane 110 can be less than 0.015" or more than 0.125". A positional membrane 110 having a larger thickness R will be less compliant (than a positional membrane with a smaller thickness). In turn this will cause the positional membrane 110 to exert more force on the endoscope 102 to secure the tip protector 100 to the endoscope (as suited for smaller diameter endoscopes). In another example, the positional membrane 110 can have a smaller thickness R for protecting an endoscope having a larger diameter. Here, the smaller thickness will make the positional membrane 110 more compliant (as compared to a positional membrane with a larger thickness) to better receive the endoscope. In addition to adjusting the thickness R of the positional membrane, the geometry of the positional membrane 110 can also be adjusted in various ways to accommodate different size endoscopes. For example, in some embodiments, the dimensions of the slots can 217 can be adjusted based on the size of the endoscope 102.

The positional membrane 110 can be connected to the cage 104 at any position that allows a distal tip 101 of an endoscope 102 to be disposed within the channel 112. In other words, the positional membrane 110 can be connected at any position along the length L of the cage 104. In the illustrated embodiment, the positional membrane 110 is connected to the cage 104 at a position A. In one exemplary embodiment, the positional membrane 110 is positioned at a center point along the length L of the cage 104. In another embodiment, the positional membrane 110 is positioned at the proximal end 106 of the cage 104. In yet another embodiment, the positional membrane 100 extends from the proximal end 106 of the cage 104 such that the positional membrane is not positioned along the length L of the cage. The positional membrane 110 can be connected to the cage 104 by any suitable means, such as, for example, being integrally molded, over-molded, ultrasonically welded, bonded or mechanically locked, etc. In certain embodiments, the cage 104 and the positional membrane 110 form a single piece that is manufactured using injection molding or other suitable means.

In various embodiments, the tip protector 100 is configured such that either end 106 or 108 can be the proximal or the distal end of the tip protector. That is, the positional membrane 110 is positioned within the channel 112 such that the distal tip of the endoscope 102 can be inserted at either end of the tip protector. When the positional membrane 110 is disposed along the length L of the cage 104 at a center point (or relatively close to a center point) of the cage 104, the cage 104 and the channel 112 take substantially the same form on each side of the positional membrane 110. In addition, the positional membrane 110 can be made of a material that allows the positional membrane to flex toward either side of the cage 104. In these embodiments, the distal tip 101 of an endoscope 102 can be inserted in either end of the tip protector 100 to secure the tip protector to the endoscope. Being able to insert an endoscope 102 into either end of the tip protector 100 is advantageous because it provides a simpler means of attaching the tip protector to the endoscope.

The portion of the channel 112 that extends between the positional membrane 110 and the distal end 108 of the tip protector 100 has a length X, and the distal tip 101 of the endoscope 102 is disposed in this portion of the channel when the tip protector 100 is attached to the endoscope. A longer distance X allows for the contact point between the positional membrane 110 and the endoscope 102 to be a further distance away from the distal tip 101 of the endoscope 102 as compared to a shorter distance X. This is advantageous because it may prevent residual fluids from contacting the distal tip of the endoscope and may help to eliminate trapping of water. In certain embodiments, the distance X between the positional membrane 110 and the distal end 108 of cage 104 can be between about 0.5" and about 8".

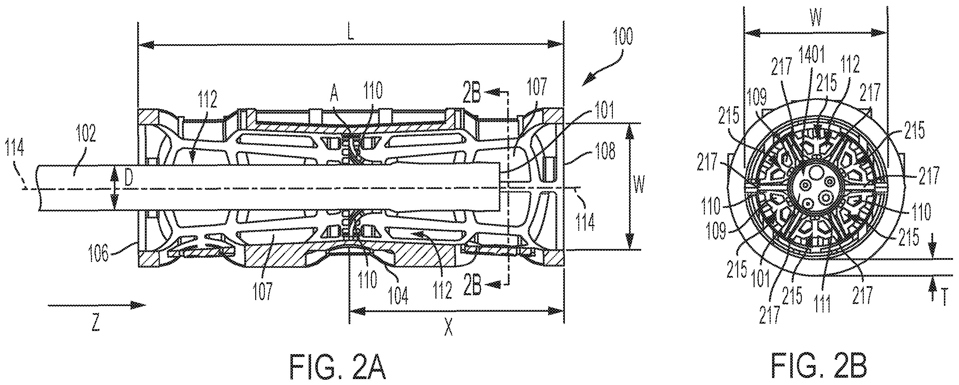

Referring to FIGS. 2A and 2B, another exemplary embodiment of a tip protector 100 is provided. The tip protector 100 includes a cage 104 and at least one positional membrane 110. The cage 104 has a plurality of holes 107 disposed between its proximal end 106 and distal end 108 that allow for air flow. The cage 104 defines a channel 112 that extends along its length L. In the illustrated embodiment, the cross-sectional shape of the channel 112 and the cage 104 is a circular shape (as shown in FIG. 2B), but can take any other suitable shape, such as a polygonal or an oval. The proximal end 106 and/or the distal end 108 of the cage 104 can be open such that air flow can enter the channel 112 to facilitate drying of any residual moisture on an attached endoscope. In certain embodiments, the tip protector 100 is configured such that a center of the channel 112, a center of the proximal end 106, and a center of the distal end 108 are aligned on an axis 114.

When the tip protector 100 is attached to the endoscope 102, the distal tip 101 of the endoscope is disposed within channel 112. The cage 104 prevents outside objects from contacting the distal tip 101 of the endoscope 102 during storage or transportation. In certain embodiments, the cage 104 is made from a soft material such as, for example, thermoplastic elastomer (TPE), flexible PVC, Polyurethane, Silicone, other polymeric material, paper-based material, etc. The length L of the cage can be, for example, between about 1" and about 9". In certain embodiments, the width W between the inner surfaces of the cage 104 can be between

about 0.125" and about 2.75" greater than the diameter D of the endoscope 102 it is meant to protect. In certain embodiments, the width W between the inner surfaces of the cage 104 can be between about 0.425" and about 3.25". The above-mentioned ranges for the width W between the inner surfaces of the cage 104 are exemplary, and it should be understood that the cage 104 can be manufactured to have any suitable width W based on the endoscope (or other medical device) that the cage 104 is meant to protect. The thickness T of the cage 104 can be between about 0.020" and about 0.150".

In the illustrated embodiment, the tip protector 100 has one positional membrane 110 but it should be understood that the tip protector can have more than one such membrane. The positional membrane 110 is configured to contact the endoscope 102 and secure the tip protector 100 to the endoscope. In certain embodiments, the positional membrane 110 is configured to attach the tip protector 100 to the endoscope 102 such that there is no contact between the cage 104 and the endoscope 102 during storage. In some situations, the endoscope 102 can be moved within the cage 104 such that there is minimal contact between the cage 104 and the endoscope (rather than no contact), but the tip protector 100 can be configured such that there is no contact between the cage 104 and the endoscope 102 when the endoscope is properly secured to the tip protector by the positional membrane 110. As described above, residual moisture on an endoscope often resides at the connection between an endoscope and a tip protector, and the residual moisture can result in bacteria or biofilm growth. As a result, the tip protector 100 described herein is advantageous because it reduces the contact points between the endoscope 102 and the tip protector 100, which reduces the areas in which residual moisture can reside. It should be appreciated that minimal contact may be any contact (e.g., incidental contact) that does not affect the functionality of the cage 104.

The positional membrane 110 includes a main opening 111 configured to receive the endoscope and a plurality of air flow openings 109. In certain embodiments, the main opening 111 of the positional membrane is aligned with the axis 114 that extends between the proximal end 106 and the distal end 108 of the cage 104.

The positional membrane 110 is flexible in that it is movable between a normal position and an attached position. In some embodiments, when the positional membrane 110 is in the normal position, the positional membrane is substantially planar, and, when the positional

membrane 110 moves from the normal position to the attached position, the positional membrane 110 flexes such that the main opening 111 expands to receive an endoscope. In the present invention, the positional membrane 110 includes a plurality of web members 215 that surround the main opening 111. Each of the web members 215 are separated from each other by slot 217 such that the web member can move independently of each other. The web members 215 include air flow openings 109 that allow for air flow through the positional membrane 110 such that the openings 109 allow any residual moisture on an attached endoscope to dry and/or exit during storage. The slots 217 also allow the main opening 111 to expand.

While the flexibility of the positional membrane 110 allows the main opening 111 to expand as needed, the positional membrane is configured to remain or move back to the normal position when it is not in use. When the tip protector 100 is attached to an endoscope 102, the ability of the positional membrane to move back to the normal protection position provides a force on the endoscope 102 that causes the tip protector to be secured to the endoscope. In some instances, repositioning the endoscope 102 within the tip protector could result in the positional membrane 110 flexing toward either the proximal end 106 and the distal end 108. The direction that the positional membrane 110 flexes (e.g., toward the proximal end 106 or the distal end 108) does not affect the ability of the positional membrane 110 to secure the tip protector 100 to the endoscope 102. That is, regardless of whether the positional membrane is flexing towards the proximal 106 or the distal end 108, the positional membrane 110 will desire to move back to the normal position, which will create a force on the endoscope that causes the tip protector to be secured to the endoscope.

The positional membrane 110 can be made of any suitable flexible material that is capable of securing the tip protector 100 to an endoscope, such as, for example, thermoplastic elastomer (TPE), flexible PVC, Polyurethane, Silicone, or other polymeric material, or paper- based material, etc. The material of the positional membrane 110 can be selected based on the characteristics of the medical device (e.g., endoscope 102) that is being protected. For example, the positional membrane 110 can be made of a harder material (e.g., a less compliant material) for protecting an endoscope 102 having a smaller diameter, and the positional membrane 110 can be made of a softer material (e.g., a more compliant material) for protecting an endoscope 102 having a larger diameter.

In certain embodiments, the thickness R of the positional membrane 110 can be between about 0.015" and about 0.125". In other embodiments, the thickness R of the positional membrane 110 can be less than 0.015" or more than 0.125". The thickness R of the positional membrane 110 can be selected based on the characteristics of the medical device (e.g., endoscope 102) that is being protected. For example, the positional membrane 110 can have a larger thickness R for protecting an endoscope 102 having a smaller diameter because the larger thickness will make the positional membrane less compliant (as compared to a positional membrane with a smaller thickness), which will cause the positional membrane 110 to exert more force on the endoscope 102 to secure the tip protector 100 to the endoscope. In another example, the positional membrane 110 can have a smaller thickness R for protecting an endoscope having a larger diameter because the smaller thickness will make the positional membrane more compliant (as compared to a positional membrane with a larger thickness), which will allow the positional membrane to receive the endoscope.

The positional membrane 110 can be connected at any position along the length L of the cage 104. In the illustrated embodiment, the positional membrane 110 is connected to the membrane at a position A along the length L of the cage 104. In one exemplary embodiment, the positional membrane 110 is positioned at a center point of the cage 104. In another exemplary embodiment, the positional membrane 110 is positioned at the proximal end 106 of the cage 104. In yet another embodiment, the positional membrane 100 extends from the proximal end 106 of the cage 104 such that it is not positioned along the length L of the cage. The positional membrane 110 can be connected to the cage 104 by any suitable means, such as, for example, being integrally molded, over-molded, ultrasonically welded, bonded or mechanically locked, etc. In certain embodiments, the cage 104 and the positional membrane 110 form a single piece that is manufactured using injection molding or any other suitable means. As explained above, the tip protector 100 is configured such that either end 106, 108 can be the proximal end or the distal end of the tip protector. That is, the positional membrane 110 is positioned within the channel 112 such that the distal tip of the endoscope 102 can be inserted at either end 106, 108 of the tip protector 100.

In certain embodiments, the portion of the channel 112 that extends between the positional membrane 110 and the distal end 108 has a length X, and the distal tip 101 of the endoscope 102 is disposed in this portion of the channel when the tip protector 100 is attached to the endoscope. It is advantageous to have a distance X that allows the contact point between the positional membrane 110 and the endoscope 102 to be further away from the distal tip 101 of the endoscope in order to prevent residual fluid from contacting the distal tip of the endoscope. In certain embodiments, the distance X between the positional membrane 110 and the distal end 108 can be between about 0.5" and about 8".

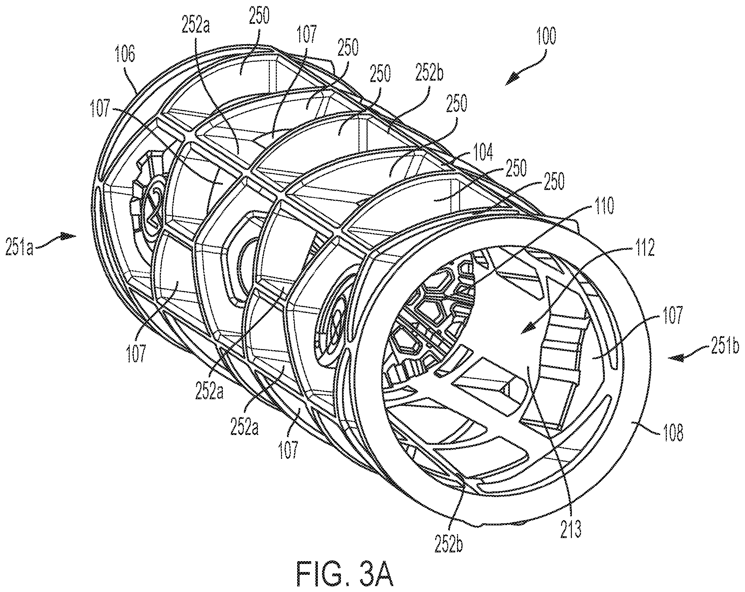

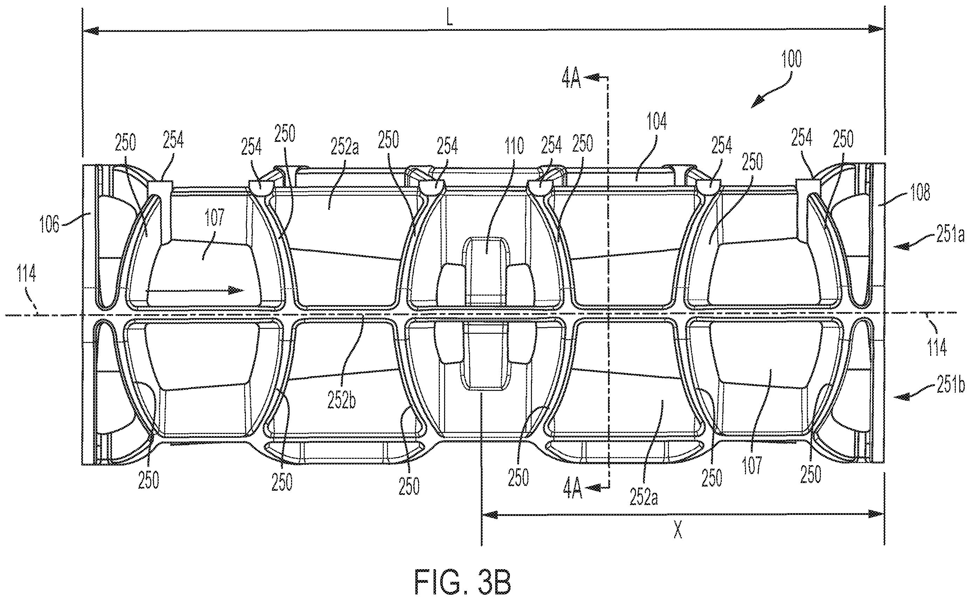

Referring to FIGS. 3A-3E, another embodiment of a tip protector 100 includes a cage 104 and a positional membrane 110. The cage 104 has a plurality of air flow holes 107 disposed between a proximal end 106 and a distal end 108. In addition, the cage 104 includes a plurality of ribs 250 connected to each other by a plurality of walls 252a, 252b, in which the ribs 250 and walls 252a 252b at least partially define the holes 107. And in some embodiments the ribs 250 may be angled to promote the elimination of residual moisture. The cage 104 defines a channel 112 that extends along the length L of the cage. In the illustrated embodiment, the cross- sectional shape of the channel 112 and the cage 104 is a circular shape (as shown in FIG. 3B). The proximal end 106 and the distal end 108 of the cage 104 are open such that air flow can enter the channel 112 to dry residual moisture on the endoscope and to allow residual moisture to exit the tip protector. In addition, the channel 112, the center of the proximal end 106, and the center of the distal end 108 are aligned on an axis 114.

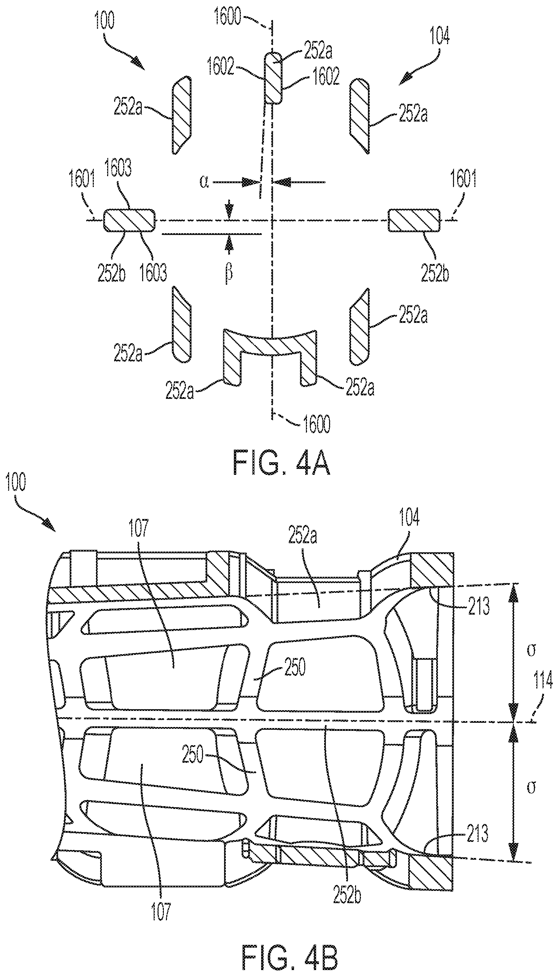

In some embodiments, the tip protector 100 can be made using injection molding. For example, referring to FIGS. 3A and 3B, the tip protector 100 can include a first portion 251a and a second portion 251b that are connected during the injection molding process to create the cage 104. Each portion 251a, 251b can include ribs 250 and walls 252a, and the connection between the first portion 251a and the second portion 251b forms walls 252b. In certain embodiments, referring to FIG. 4A, each side of the walls 252a can have a draft angle α that makes it easier to remove the cage 104 from a mold. That is, FIG. 4A shows an axis 1600 extending through a center of the wall 252a and a draft angle α exists between the axis 1600 and the sides 1602 of the wall 252a. The draft angle α can be, for example, between about 0.5° and about 5°, and, as a result, the included angle between the sides 1602 can be between about 1° and about 10°. In some embodiments, the draft angle α can be between about 0.5° and about 3°, such as between about 0.5° and about 2°, such as about 1°.

Still referring to FIG. 4A, in certain embodiments, each side of walls 252b can have a draft angle β. Draft angle β exists between the sides 1603 of the wall 252B and the parting line 1601 that separates the first portion 251a (FIGS. 3A-3B) and the second portion 251B (FIGS. 3A-3B) of the cage 104. The draft angle β can be, for example, between about 5° and about 30°, and, as a result, the included angle between the sides 1603 can be between about 10° and about 60°. In some embodiments, the draft angle β can be between about 5° and about 20°, such as between about 5° and about 15°, such as about 10°.

Referring to FIGS. 3A-3B and 4B, in certain embodiments, the inner wall 213 of the cage 104 is tapered from the location of the positional membrane 110 to the proximal end 106 and/or distal end 108 of the cage 104. That is, referring to FIG. 4B, an axis 114 is shown through a center of the tip protector 100, and the inner wall 213 of the cage is tapered such that a draft angle θ exists between the axis 114 and the inner wall 213 of the cage 104. The draft angle θ can be, for example, between about 1° and about 5°, and, as a result, the included angle between the opposite portions of the inner wall 213 can be between about 2° and about 10°. In some embodiments, the draft angle θ can be between about 2° and about 4°, such as about 3°.

Referring to FIG. 3B, the cage 104 can include a plurality of pins 254 configured to assist in removing the tip protector 100 from a molding. The pins 254 can be located on any portion of the cage 104 and can take any suitable form that allows a force to be provided to the pins to remove the tip protector from the molding.

In certain embodiments, the cage 104 is made from a soft material, such as, for example, thermoplastic elastomer (TPE), flexible PVC, Polyurethane, Silicone, or other polymeric material, or paper-based material, etc. The length L of the cage can be, for example, between about 0.5" and about 8". The width W between the inner surfaces of the cage 104 can be between about 0.020" and about 0.350". The thickness T of the cage 104 can be between about 0.20" and about 0.350".

In the present invention, the positional membrane 110 includes a main opening 111 that is configured to receive an endoscope and a plurality of web members 215 that surround the main opening 111. Each of the web members 215 is separated by a slot 217 such that the web members can move independently of each other. The web members 215 include openings 109 that allow for air flow through the positional membrane 110 such that any residual moisture on an attached endoscope can dry or exit during storage which prevents residual moisture from residing at the contact point between the positional membrane 110 and the endoscope. In certain embodiments, the main opening 111 of the positional membrane is aligned with the axis 114 that extends between the proximal end 106 and the distal end 108 of the cage 104.

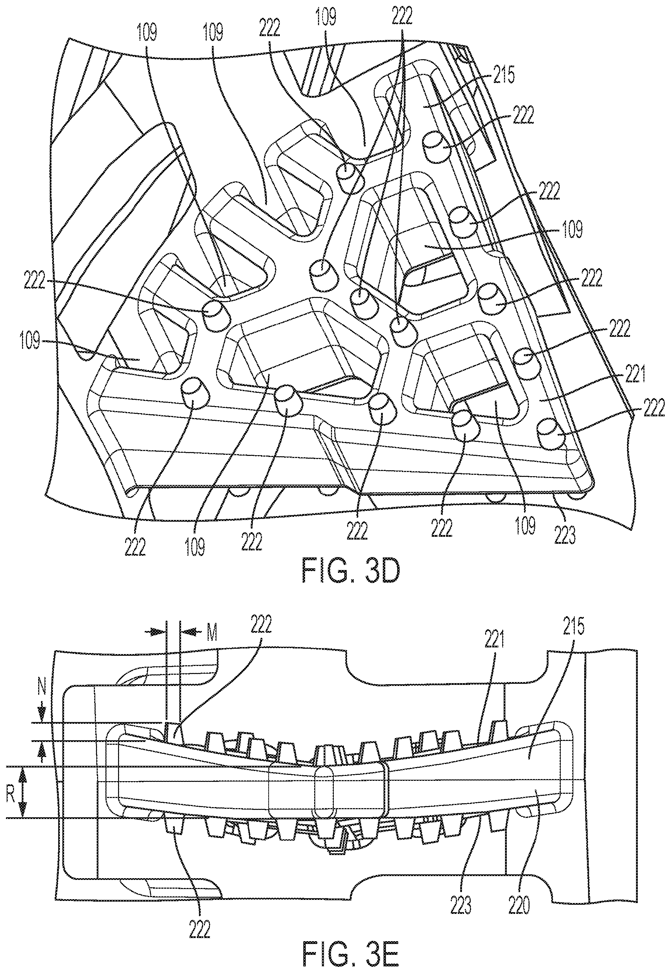

FIGS. 3D and 3E illustrate an exemplary embodiment of a web member 215 that includes a body member 220, a plurality of openings 109, and a plurality of engagement members 222. The engagement members 222 are configured to engage an endoscope when the tip protector 100 is attached to the endoscope such that minimal contact exists between the positional membrane 110 and the endoscope. In the illustrated embodiment, the engagement members 222 take the form of nodules that extend from the body member 220. The contact surface of the engagement members 220 can have a width M between about 0.005" and about 0.060", and the engagement members 222 can extend from the body member 220 a distance N between about 0.010" and about 0.100". It should be appreciated that the nodules extending from the body member 220 may reduce a surface contact area of the endoscope. In certain embodiments, the positional membrane has a first side 221 that faces towards the proximal end 106 (FIG. 3B) of the tip protector 100 and a second side 223 that faces towards a distal end 108 (FIG. 3B) of the tip protector 100, and each side 221, 223 of the positional membrane 110 includes a plurality of engagement members 222. Having engagement members 222 on each side 221, 223 of the positional membrane 110 allows an endoscope to be inserted into either end 106, 108 of the tip protector 100 to attach the tip protector to the endoscope.

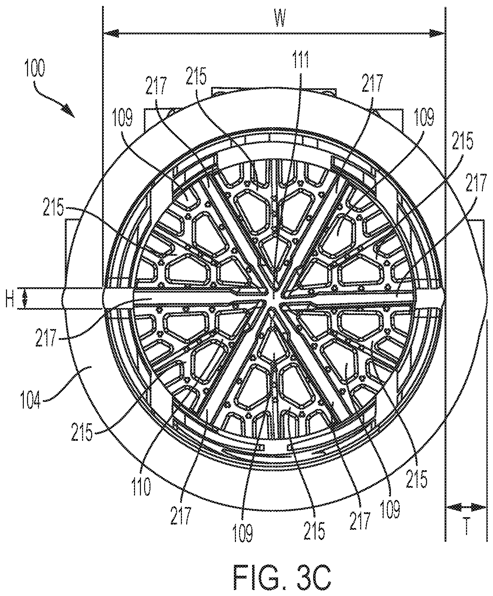

Referring to FIGS. 3C-3E, the web members 215 can take any form that surrounds the main opening 111 of the positional membrane 110, that allows the main opening 111 to expand to receive an endoscope, and that is configured to secure the tip protector 100 to an endoscope. In the illustrated embodiment, each of the web members 215 takes the form of a sector of a circle (e.g., the shape of a slice of pizza). That is, each web member 215 has a curved edge that conforms with the shape of the inner wall 213 of the cage 104 and a pair of substantially straight edges that extends toward the main opening 110. In other embodiments, the edge of the web member 215 that connects to the inner wall 213 of the cage 104 may need to take a non-curved form to connect to the inner wall 213.

Referring to FIGS. 3A-3E, the positional membrane 110 is flexible such that the positional membrane is movable between normal and attached positions. In some embodiments, for example, when the positional membrane 110 is in the normal position, the positional membrane 110 may be curved such that it may be wider at the base. When the positional membrane 110 moves from the normal position to the attached position, the positional membrane 110 flexes such that the main opening 111 expands to receive an endoscope. Referring to FIG. 3C, the main opening 111 has a width H when the positional membrane is in the normal position. The width H can be, for example, between about 0.040" and about 0.500". In certain embodiments, the main opening 111 is capable of moving to an expanded position such that the main opening has an expanded width of between about 0.050" and about 0.550" (e.g., such that it can receive an endoscope In certain embodiments, the ratio between the maximum expanded width of the main opening 111 and the width H of the main opening (when the positional membrane 110 is in the normal position) is between about 1 to 1 and about 17.5 to 1.

The positional membrane 110 is configured to remain or move back to the normal position when it is not in use. As a result, when the tip protector 100 is attached to an endoscope, the positional membrane provides a force on the endoscope that causes the tip protector to be secured to the endoscope. The positional membrane 110 can be made of any suitable flexible material that is capable of securing the tip protector 100 to an endoscope, such as, for example, thermoplastic elastomer (TPE), flexible PVC, Polyurethane, Silicone, or other polymeric material, or even paper-based material, etc. The thickness R (FIG. 3E) of the positional membrane 110 can be, for example, between about 0.20" and about 0.350".

The positional membrane 110 can be connected at any position along the length L of the cage 104, or it can extend from the proximal end 106 of the cage 104. In the illustrated embodiment, the positional membrane 110 is positioned at a center point of the cage 104. The positional membrane 110 can be connected to the cage 104 by any suitable means, such as, for example, being integrally molded, over-molded, ultrasonically welded, bonded or mechanically locked, etc. In certain embodiments, the cage 104 and the positional membrane 110 form a single piece that is manufactured using injection molding or any other suitable means.

In some embodiments, the cage 104 and the channel 112 can take substantially the same form on each side of the positional membrane 110, and the positional membrane 110 can be made of a material that allows the positional membrane to flex toward either side 106, 108 of the cage 104. In addition, as shown in FIGS. 3D-3E, each side 221, 223 of the positional membrane 110 can include engagement members 222 for engaging an endoscope. In these embodiments, the distal tip of an endoscope can be inserted in either end 106, 108 of the tip protector 100 to secure the tip protector to the endoscope. In certain embodiments, the portion of the channel 112 that extends between the positional membrane 110 and the distal end 108 of the cage 104 has a length X, and the distal tip of the endoscope is disposed in this portion of channel when the tip protector 100 is attached to the endoscope. The distance X between the positional membrane 110 and the distal end 108 of cage 104 can be between about 0.5" and about 8".

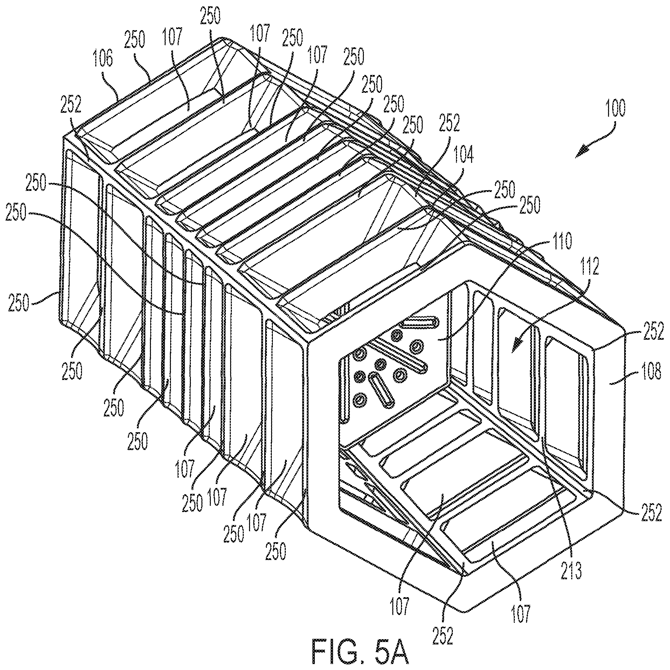

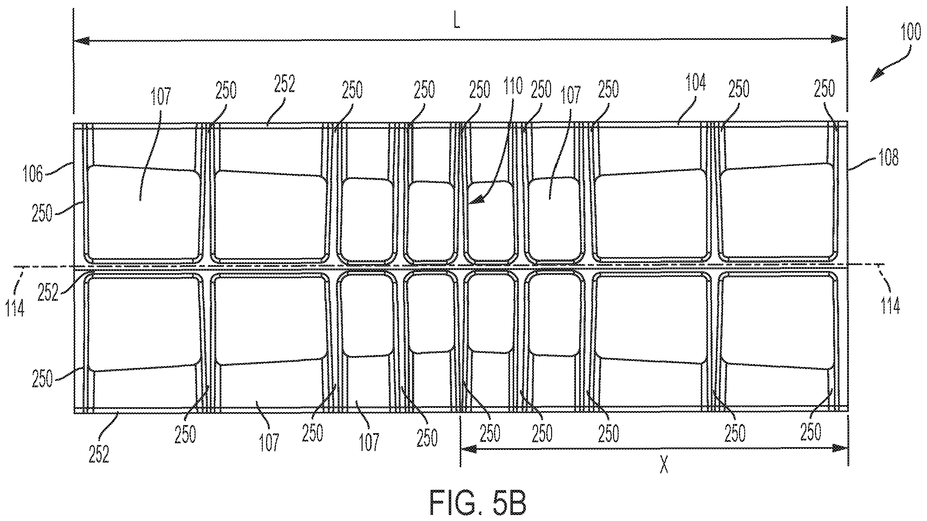

Referring to FIGS. 5A-5C, another embodiment of a tip protector 100 is shown having a cage 104 and a positional membrane 110. The cage 104 has a plurality of holes 107 disposed between a proximal end 106 and a distal end 108. In addition, the cage 104 includes a plurality of ribs 250 connected to each other by a plurality of walls 252, in which the ribs 250 and walls 252 at least partially define the holes 107. The cage 104 defines a channel 112 that extends along its length L. In the illustrated embodiment, the cross-sectional shape of the channel 112 and the cage 104 is a hexagonal shape (as shown in FIG. 5B). In other embodiments, the channel 112 and cage 104 can take any other suitable shape, such as a circular shape, a polygonal shape, an oval shape, or the like. The proximal end 106 and the distal end 108 of the cage 104 are open such that air flow can enter the channel 112 to dry residual moisture on the endoscope and to allow residual moisture to exit the tip protector. In addition, in certain embodiments, the channel 112, the center of the proximal end 106, and the center of the distal end 108 are aligned on an axis 114.

In certain embodiments, the tip protector 100 can be made using injection molding. For example, similar to the embodiment shown in FIG. 3A-3E and 4A-4B, the tip protector 100 can include two or more portions that are connected during the injection molding process to create the cage 104. Each portion can include ribs 250 and walls 252. In certain embodiments, the cage 104 of the tip protector 100 can be configured to assist in the removal of the tip protector from a molding. For example, the walls 252 can have a draft angle on each side of the walls 252 (e.g., the draft angle α shown in FIG. 4A and described above). In addition, the walls 252 at the connection between the two or more portions can have a draft angle on each side of the wall 252 (e.g., the draft angle β shown in FIG. 4A as described above). Referring to FIGS. 5A-5B, in certain embodiments, the inner wall 213 of the cage 104 is tapered from the location of the positional membrane 110 to the proximal end 106 and/or distal end 108 of the cage 104. That is, the inner wall 213 of the cage 104 is tapered such that a draft angle exists between the axis 114 and the inner wall 213 of the cage 104 (e.g., the draft angle θ shown in FIG. 4B and described above). In certain embodiments, the cage 104 includes a plurality of pins (e.g., pins 254 shown in FIGS. 3B) that are configured to assist in removing the tip protector 100 from a mold.

When the tip protector 100 is attached to an endoscope, the distal tip of the endoscope is disposed within the channel 112 such that the cage 104 surrounds the distal tip to protect it from being damaged. In certain embodiments, the cage 104 is made from a soft material, such as, for example, thermoplastic elastomer (TPE), flexible PVC, Polyurethane, Silicone, or other polymeric material, or paper-based material, etc. The length L of the cage can be, for example, between about 1" and about 9". The width W between the outer surfaces of the cage 104 can be between about 0.125" and about 1.5" greater than the outside diameter of the endoscope it is designed to protect. The thickness T of the cage 104 can be between about 0.020" and about 0.350".

In the illustrated embodiment, the positional membrane 110 includes a main opening 111 that is configured to receive an endoscope, a plurality of air flow openings 109, and a plurality of slots 325 that extend into the main opening 111. The air flow openings 109 allow any residual moisture on an attached endoscope to dry or exit during storage. The main opening 111 can be aligned with the axis 114 that extends between the proximal end 106 and the distal end 108 of the cage 104. The slots 325 are positioned to allow the main opening 111 to expand. That is, because the slots 325 extend into the main opening 111, the main opening 111 can expand to a larger size when an endoscope is inserted into the positional membrane 110. In certain embodiments, the positional membrane 110 can include engagement members (e.g., the engagement members 222 shown in FIGS. 3D-3E and described above) that are configured to engage an endoscope such that minimal contact exists between the positional membrane 110 and the endoscope.

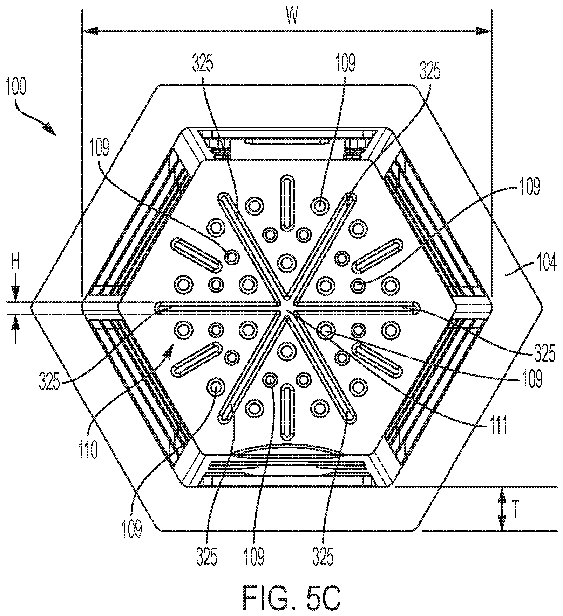

Referring to FIG. 5C, the main opening 111 has a width H when the positional membrane is in a normal position. The width H can be, for example, between about 0.040" and about 0.500". In certain embodiments, the positional membrane 110 flexes such that the main opening 111 expands to an expanded width of between about 0.050" and about 0.550". In certain embodiments, the ratio between the maximum expanded width of the main opening 111 and the width H of the main opening (when the positional membrane 110 is in the normal position) is between about is between about 1 to 1 and about 17.5 to 1.

While the flexibility of the positional membrane 110 allows the main opening 111 to expand as needed, the positional membrane is configured to remain or move back to the normal position when it is not in use. Because the positional membrane 110 desires to move back to its normal position, when the tip protector 100 is attached to an endoscope, the positional membrane provides a force on the endoscope that causes the tip protector to be secured to the endoscope. The positional membrane 110 can be made of any suitable flexible material that is capable of securing the tip protector 100 to an endoscope, such as, for example, thermoplastic elastomer (TPE), flexible PVC, Polyurethane, Silicone, or other polymeric material, or paper- based material, etc. The thickness of the positional membrane 110 can be, for example, between about 0.020 and about 0.350".

The positional membrane 110 can be connected to the cage 104 at any position that allows a distal tip of an endoscope to be disposed within the channel 112 when the tip protector

100 is connected to the endoscope for example, the positional membrane 110 can be positioned relative to the cage 104 in any manner described in the present application. In the illustrated embodiment, the positional membrane 110 is positioned at a center point along the length L of the cage 104. The positional membrane 110 can be connected to the cage 104 by any suitable means, such as, for example, integrally molded, over-molded, ultrasonically welded, bonded or mechanically locked, etc. In certain embodiments, the cage 104 and the positional membrane 110 form a single piece that is manufactured using injection molding or any other suitable means.

In certain embodiments, the portion of the channel 112 that extends between the positional membrane 110 and the distal end 108 of the cage 104 has a length X, and the distal tip of the endoscope is disposed in this portion of channel when the tip protector 100 is attached to the endoscope. In certain embodiments, the distance X between the positional membrane 110 and the distal end 108 of cage 104 is between about 0.5" and about 8".

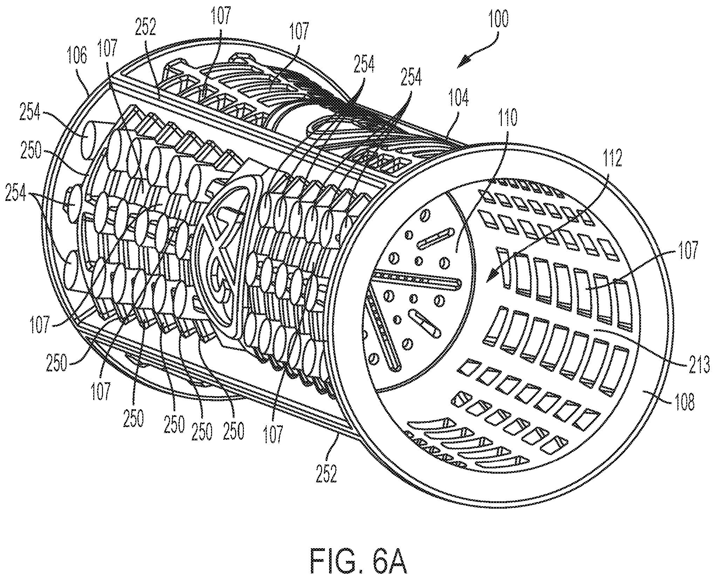

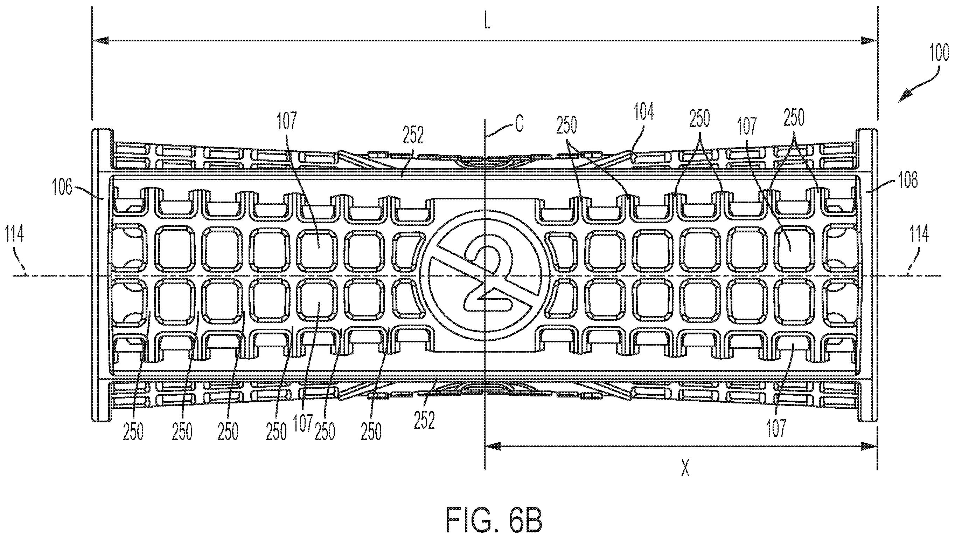

Referring to FIGS. 6A-6C, another embodiment of a tip protector 100 includes a cage 104 and a positional membrane 110. The cage 104 has a plurality of holes 107 disposed between a proximal end 106 and a distal end 108. The holes 107 allow for air flow, such that any residual moisture on an attached endoscope can dry or exit during storage. In addition, the cage 104 includes a plurality of ribs 250 connected to each other by a plurality of walls 252, in which the ribs 250 and walls 252 at least partially define the holes 107. The cage 104 defines a channel 112 that extends along the length L of the cage 104 between the proximal end 106 and the distal end 108. In the illustrated embodiment, the cross-sectional shape of the channel 112 and the cage 104 is a circular shape (as shown in FIG. 6B). In other embodiments, the channel 112 and cage 104 can take any other suitable shape, such as a polygonal shape, an oval shape, or the like. In certain embodiments, as shown in FIGS. 6A-6B, a channel 112 tapers from the ends 106, 108 to the center of the cage 104 (shown by center line C). In the illustrated embodiment, the diameter (or width) of the channel 112 at the ends is larger than the diameter (or width) of the channel 112 at the center line C, which is advantageous because the larger openings allow more air flow through the channel 112. In some embodiments, a draft angle exists between an axis 114 and the inner wall 213 of the cage 104 (e.g., draft angle θ shown in Fig. 4B and described above). The ratio of the diameter (or width) at the ends 106, 108 to the diameter (or width) at the center line C can be between about 1 to 1 and about 10 to 1. In other embodiments, the diameter (or width) of the channel 112 at the ends can be smaller than the diameter (or width) of the channel 112 at the center line C. In some embodiments, the diameter (or width) of the channel remains constant throughout the length L of the cage 104. The proximal end 106 and the distal end 108 of the cage 104 are open such that air flow can enter the channel 112 to dry residual moisture on the endoscope and to allow residual moisture to exit the tip protector. In addition, in certain embodiments, the channel 112 and the centers of the end 100, 108 are aligned on an axis 114.

In certain embodiments, the tip protector 100 can be made using injection molding. For example, similar to the embodiment shown in FIG.3-3E and 4A-4B, the tip protector 100 can include two or more portions that are connected during the injection molding process to create the cage 104 and include ribs 250 and walls 252. In certain embodiments, the cage 104 of the tip protector 100 can be configured to assist in the removal of the tip protector from a molding. For example, the walls 252 can have a draft angle on each side of the walls 252 (e.g., the draft angle α shown in FIG. 4A and described above). In certain embodiments, the cage 104 includes a plurality of pins 254 that are configured to assist in removing the tip protector 100 from a molding.

When the tip protector 100 is attached to an endoscope, the cage 104 prevents any outside object from contacting the distal tip of the endoscope during storage or transportation. In certain embodiments, the cage 104 is made from a soft material such as, for example, thermoplastic elastomer (TPE), flexible PVC, Polyurethane, Silicone, or other polymeric material, or paper-based material, etc. The soft material prevents damage to the distal tip of the endoscope if the inner wall 213 of the cage 104 contacts the distal tip. The length L of the cage 104 can be, for example, between about 1" and about 9". The width W between the inner surfaces of the cage 104 can be between about 0.20" and about 0.350". The thickness T of the cage 104 can be between about 0.020" and about 0.350".

The positional membrane 110 is connected to the cage 104 and configured to attach the tip protector 100 to an endoscope such that the only contact between the endoscope and the tip protector is between the positional membrane and the endoscope. That is, the positional membrane 110 is configured to attach the tip protector 100 to an endoscope such that the endoscope does not contact any portion of the cage 104 during storage of the endoscope. Accordingly, the tip protector 100 is advantageous because it reduces the contact points between the endoscope and the tip protector 100, which reduces the areas in which residual moisture can reside and cause bacterial build up.

In the illustrated embodiment, the positional membrane 110 includes a main opening 111 that is configured to receive an endoscope, a plurality of air flow openings 109, and a plurality of slots 325 that extend into the main opening 111. The openings 109 allow for air flow through the positional membrane 110 such that any residual moisture on an attached endoscope can dry or exit during storage of the endoscope. In certain embodiments, the main opening 111 of the positional membrane is aligned with the axis 114 that extends between the proximal end 106 and the distal end 108 of the cage 104. The slots 325 are positioned to allow the main opening 111 to expand. That is, because the slots 325 into the main opening 111, the main opening 111 can expand to a larger size when an endoscope is inserted into the positional membrane 110. In certain embodiments, the positional membrane 110 can include engagement members (e.g., the engagement members 222 described in FIGS. 3D-3E) that are configured to engage an endoscope when the tip protector 100 is attached to the endoscope.

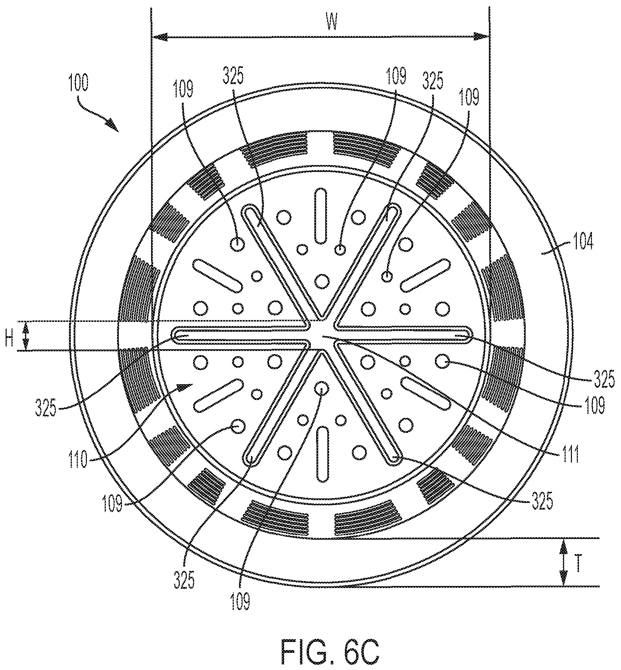

Referring to FIG. 6C, the main opening 111 has a width H when the positional membrane is a normal position. The width H can be, for example, between about 0.040" and about 0.500". In certain embodiments, the main opening 111 is capable of moving to an expanded position such that the main opening has an expanded width of between about 0.050" and about 0.550" (e.g., such that it can receive an endoscope. In certain embodiments, the ratio between the maximum expanded width of the main opening 111 and the width H of the main opening (when the positional membrane 110 is in the normal position) is between about 1 to 1 and about 17.5 to 1.

While the flexibility of the positional membrane 110 allows the main opening 111 to expand as needed, the positional membrane is configured to remain or move back to the normal position when it is not in use. As a result, when the tip protector 100 is attached to an endoscope, the positional membrane deserves to move back to the normal position, which provides a force on the endoscope that causes the tip protector to be secured to the endoscope. The positional membrane 110 can be made of any suitable flexible material that is capable of securing the tip protector 100 to an endoscope, such as, for example, thermoplastic elastomer (TPE), flexible PVC, Polyurethane, Silicone, other polymeric material, a paper-based material, etc. The thickness of the positional membrane 110 can be, for example, between about 0.20" and about 0.350".

The positional membrane 110 can be connected at any position along the length L of the cage 104, or it can extend from the proximal end of the cage 104. In the illustrated embodiment, the positional membrane 110 is positioned at a center point of the cage 104. The positional membrane 110 can be connected to the cage 104 by any suitable means, such as, for example, integrally molded, over-molded, ultrasonically welded, bonded or mechanically locked, etc. In certain embodiments, the cage 104 and the positional membrane 110 form a single piece that is manufactured using injection molding or any other suitable means.

In certain embodiments, the portion of the channel 112 that extends between the positional membrane 110 and the distal end 108 of the cage 104 has a length X, and the distal tip of the endoscope is disposed in this portion of channel when the tip protector 100 is attached to the endoscope. In some embodiments, the distance X between the positional membrane 110 and the distal end 108 of cage 104 is between about 0.5" and about 8". This distance X allows the contact point between the positional membrane 110 and the endoscope 102 to be away from the exit point of accessory channel at the scope's distal tip 101, which is advantageous because this prevents any residual moisture between the positional membrane 110 and the endoscope 102 from entering or contacting the accessory channel.

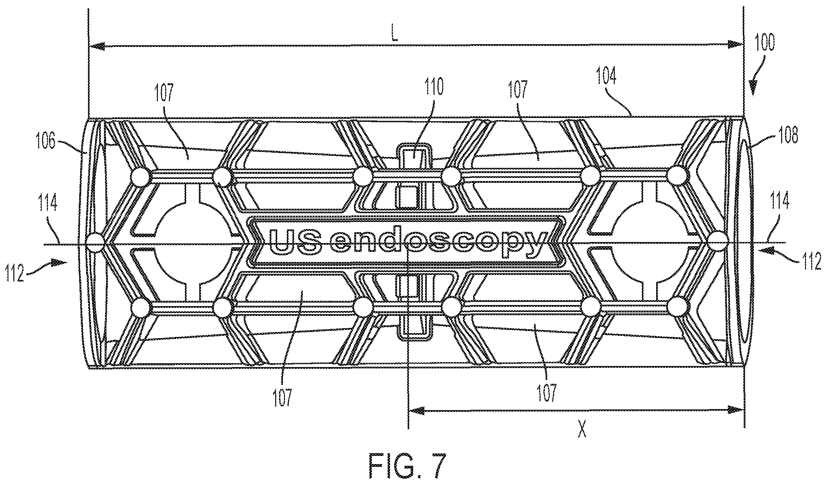

Referring to FIG. 7, another exemplary embodiment of a tip protector 100 includes a cage 104 and a positional membrane 110. The cage 104 has a plurality of holes 107 disposed between a proximal end 106 and a distal end 108, allow for air flow such that any residual moisture on an attached endoscope which can dry or exit during storage of the endoscope. The cage 104 defines a channel 112 that extends along the length L of the cage 104 between the proximal end 106 and the distal end 108. The cross-sectional shape of the channel 112 and the cage 104 can take any suitable form, such as, for example, a circular shape, a polygonal shape, an oval shape, or the like. The proximal end 106 and the distal end 108 of the cage 104 are open such that air flow can enter the channel 112 to dry residual moisture on the endoscope and to allow residual moisture to exit the tip protector. In addition, in certain embodiments, the channel 112, the center of the proximal end 106, and the center of the distal end 108 are aligned on an axis 114.

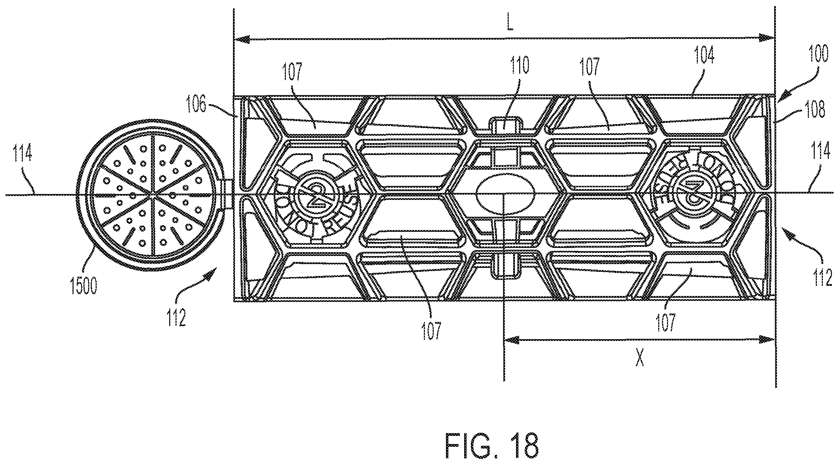

Additionally, or alternatively, embodiments of the tip protector 100 may include a cap 1500 (e.g., a flip cap) as shown in FIG 18. The tip protector 100 in this embodiment may be similar to the embodiment of FIG 7 in that it may include a cage 104 and positional membrane 110, and a plurality of holes 107 disposed between a proximal end 106 and a distal end 108, allow for air flow such that any residual moisture on an attached endoscope which can dry or exit during storage of the endoscope. The cap 1500 may be adapted or otherwise sized and/or shaped to cover (or substantially cover) at least one end (e.g., the distal end) of the tip protector 100, or more particularly, the distal end opening of the positional membrane 110.

It should be appreciated that, in the embodiment shown in FIG. 18, entry of the endoscope may be only via the proximal end of the tip projector 100 to allow for the cap 1500 to cover the distal end opening of the tip protector 100, cage 104, and/or positional membrane 110. In some embodiments, the cap 1500 may include one or more openings to permit airflow to enter through the cap 1500 for drying any residual moisture on an attached endoscope. It should be appreciated that the openings in the cap 1500 may be sized and/or shaped to restrict or otherwise limit unwanted and/or undesirable objects from entering or otherwise penetrating the tip protector 100 (or positional membrane 110) when the cap 1500 is in a closed or substantially closed position. These undesirable objects may cause damage to the endoscope or other parts operably connected thereto. In some embodiments, the cap 1500 may be integrally formed with the tip protector 100. Additionally, or alternatively, the cap 1500 may be a separate cap (i.e., part), which may be tethered or untethered to the tip protector 100. It should further be appreciated that providing the cap 1500 may assist to avoid damage to the endoscope resulting from direct contact through the center of the tip protector 100, which may thread the needle.

When the tip protector 100 is attached to an endoscope, the distal tip of the endoscope is disposed within the channel 112 of the cage 104, such that the cage 104 surrounds the distal tip to protect the distal tip from being damaged. In certain embodiments, the cage 104 is made from a soft material, such as, for example, a thermoplastic elastomer (TPE), flexible PVC, Polyurethane, Silicone, or other polymeric material, or paper-based material, etc. The length L of the cage can be, for example, between about 1" and about 9".

The positional membrane 110 is connected to the cage 104 and configured to contact an endoscope and secure the tip protector 100 to the endoscope. The positional membrane 110 can take any suitable form that is capable of securing the tip protector 100 to an endoscope such that the only contact between the endoscope and the tip protector is between the positional membrane and the tip protector. For example, the positional membrane can take any form described in the present application. The positional membrane 110 can be connected to the cage 104 at any position that allows a distal tip of an endoscope to be disposed within the channel 112 defined by the cage 104 when the tip protector 100 is connected to the endoscope. For example, the positional membrane 110 can be connected to the cage 104 described in the present application. The positional membrane 110 can be connected to the cage 104 by any suitable means, such as, for example, any means described in the present application.

In certain embodiments, the portion of the channel 112 that extends between the positional membrane 110 and the distal end 108 of the cage 104 has a length X, and the distal tip of the endoscope is disposed in this portion of channel when the tip protector 100 is attached to the endoscope. The distance X between the positional membrane 110 and the distal end 108 of cage 104 can be between about 0.5" and about 8".

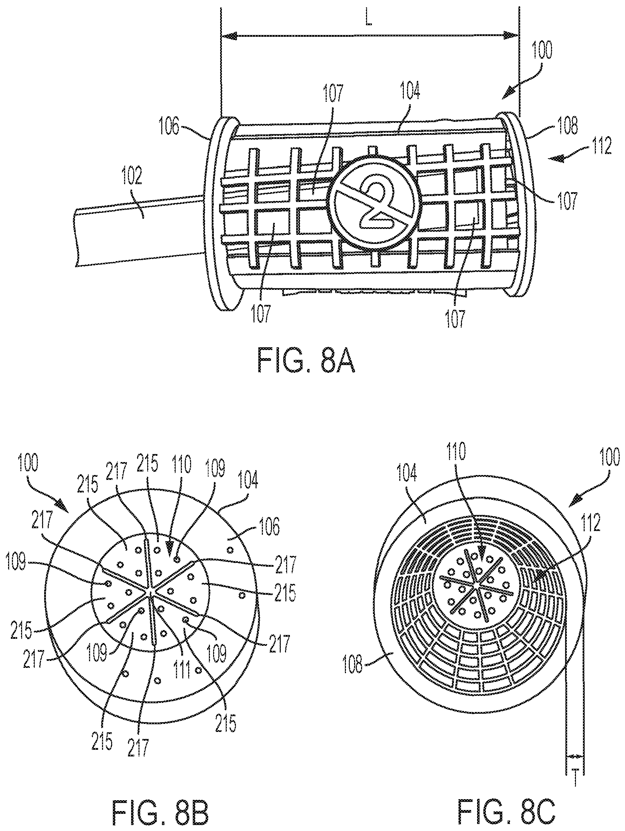

Referring to FIGS. 8A-8C, another embodiment of a tip protector 100 is shown attached to an endoscope 102. The tip protector 100 includes a cage 104 and a positional membrane 110. The cage 104 has a plurality of holes 107 disposed between a proximal end 106 and the distal end 108 that allow for air flow through the tip protector 100. The cage 104 defines a channel 112 that extends along its length L. In the illustrated embodiment, the cross- sectional shape of the channel 112 and the cage 104 is a circular shape (as shown in FIG. 8C). In other embodiments, the channel 112 and cage 104 can take any other suitable shape, such as a polygonal shape, an oval shape, or the like. In the illustrated embodiment, the distal end 108 of the cage 104 is open such that air flow can enter the channel 112 to dry residual moisture on the endoscope and to allow residual moisture to exit the tip protector. In addition, in certain embodiments, a center of the channel 112, the center of the proximal end 106, and the center of the distal end 108 are aligned.

As shown in FIG. 8A, when the tip protector 100 is attached to an endoscope, the distal tip of the endoscope is disposed within the channel 112 of the cage 104, such that the cage 104 surrounds the distal tip to protect the distal tip from being damaged. In certain embodiments, the cage 104 is made from a soft material such as, for example, thermoplastic elastomer (TPE), flexible PVC, Polyurethane, Silicone, other polymeric material, a paper-based material, etc. The length L of the cage can be, for example, between about 1" and about 9". The width W between the outer surfaces of the cage 104 can be between about 0.125" and about 1.5" greater than the outside diameter of the endoscope it is designed to protect. The thickness T of the cage 104 can be between about 0.020" and about 0.350".

Referring to FIGS. 8A and 8B, the positional membrane 110 is connected to the cage 104 at the proximal end 106 of the cage, and the positional membrane is configured to contact the endoscope 102 and secure the tip protector 100 to the endoscope 102. The positional membrane 110 is configured to attach the tip protector 100 to an endoscope such that the endoscope does not contact any portion of the cage 104 during storage of the endoscope. Accordingly, the tip protector 100 is advantageous because it reduces the contact points between the endoscope and the tip protector 100, which reduces the areas in which residual moisture can reside and cause bacterial build up.

Referring to FIG. 8B the positional membrane 110 includes a main opening 111 that is configured to receive an endoscope and a plurality of web members 215 that surround the main opening 111. The web members 215 are separated from each other by a slot 217 such that each web member can move independently of each other. The web members 215 include air flow openings 109 that allow for air flow through the positional membrane 110 such that any residual moisture on an attached endoscope can dry or exit during storage. In certain embodiments, the main opening 111 of the positional membrane is aligned on the center of the proximal opening 106, such that the main opening 111 is aligned with the center of the channel 112 and the center of the distal end 108.

The positional membrane 110 is flexible such that it can move between a normal position and an attached position When the positional membrane 110 moves from the normal position to the attached position, the positional membrane 110 flexes such that the main opening 111 expands to receive an endoscope. Although the flexibility of the positional membrane 110 allows the main opening 111 to expand as needed, the positional membrane is configured to remain or move back to the normal position when it is not in use. Because of this, when the tip protector 100 is attached to an endoscope, the positional membrane provides a force on the endoscope that causes the tip protector to be secured to the endoscope. Moreover, positional membrane 110 can be made of any suitable flexible material that is capable of securing the tip protector 100 to an endoscope, such as, for example, thermoplastic elastomer (TPE), flexible PVC, Polyurethane, Silicone, other polymeric material, a paper-based material, etc.

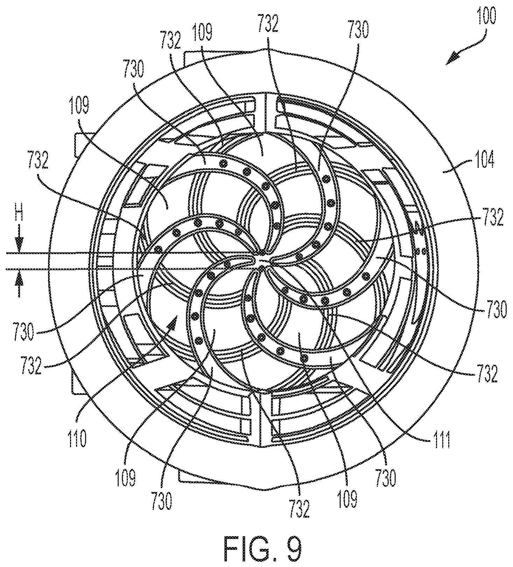

Referring to FIG. 9, an exemplary embodiment of a positional membrane 110 for a tip protector 100 includes a main opening 111 and a plurality of air flow openings 109. The positional membrane 110 is connected to the cage 104 of the tip protector 100 and is configured secure the tip protector 100 to an endoscope. The main opening 111 is configured to receive an endoscope, and the openings 109 allow for air flow through the positional membrane 110 such that any residual moisture on an attached endoscope can dry or exit during storage.

In the illustrated embodiment, the positional membrane 110 has a plurality of curved members 730 that extend from an inner wall of the cage 104, in which the ends of the curved members 730 define the main opening 111. In addition, the positional membrane has a plurality of curved supporting members 732 that intersect and connect to the curved members 730. The openings 109 are defined by both the curved members 730 and the supporting members 732. A gap exists between adjacent pairs of the curved members 730, which allows the main opening 111 to expand when it receives an endoscope. That is, because gaps exist between adjacent pairs of the curved members 730 that define the main opening 111, the ends of the curved members can move relative to each other, which allows the main opening 111 to expand to a larger size when an endoscope is inserted into the main opening.

In certain embodiments, the positional membrane 110 can include engagement members (e.g., the engagement members 222 shown in FIGS. 3D-3E and described above) that are configured to engage an endoscope when the tip protector 100 is attached to the endoscope such that minimal contact exists between the positional membrane 110 and the endoscope. The positional membrane 110 is configured to attach the tip protector 100 to an endoscope such that the endoscope does not contact any portion of the cage 104 during storage of the endoscope. The tip protector 100 is advantageous because it reduces the contact points between the endoscope and the tip protector 100, which reduces the areas in which residual moisture can reside and cause bacterial build up.

The positional membrane 110 is flexible such that the positional membrane is movable between a normal position and an attached position. In particular, the main opening 111, is configured to expand to correspond to the diameter of an endoscope. Referring to FIG. 9, the main opening 111 has a width H when in the normal position. The width H can be, for example, between about 0.040" and about 0.500". The main opening 111 is configured to expand to an expanded width of between about 0.050" and about 0.550" In certain embodiments, the ratio between the maximum expanded width of the main opening 111 and the width H of the main opening (when the positional membrane 110 is in the normal position) is between about is between about 1 to 1 and about 17.5 to 1.

While the flexibility of the positional membrane 110 allows the main opening 111 to expand as needed, the positional membrane is configured to remain or move back to the normal position when it is not in use. Because the positional membrane 110 desires to move back to its normal position, when the tip protector 100 is attached to an endoscope, the positional membrane provides a force on the endoscope that causes the tip protector to be secured to the endoscope. The positional membrane 110 can be made of any suitable flexible material that is capable of securing the tip protector 100 to an endoscope, such as, for example, thermoplastic elastomer (TPE), flexible PVC, Polyurethane, Silicone, other polymeric material, a paper-based material, etc.

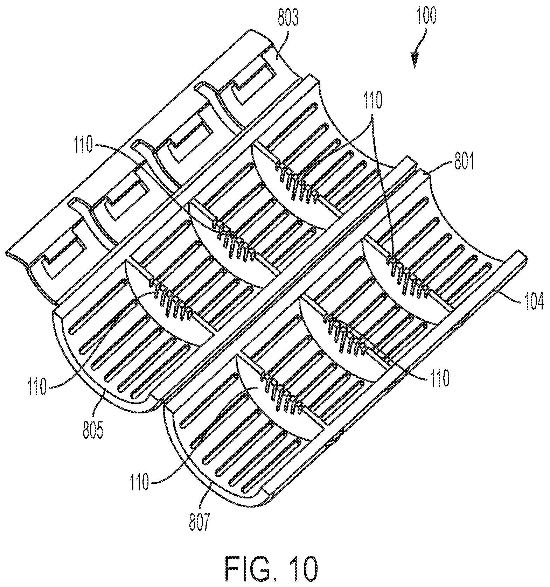

FIGS. 10 through 12B illustrate exemplary embodiments of tip protectors 100 that include one or more hinges 801 such that the tip protector is movable between an open position and a closed position. Referring to FIG. 10, the tip protector 100 includes a cage 104 having a hinge 801 and a latch member 803. The hinge 801 separates the tip protector into a first portion 805 and a second portion 807. In the illustrated embodiment, the tip protector 100 includes three positional membranes 110, in which a half of each of the membranes 110 is connected to a first portion 805 of the cage, and the other half of each of the membranes 110 is connected to the second portion 807 of the cage. In other embodiments, the tip protector 100 can have less than or more than three positional membranes 110. The positional membranes 110 can take any suitable form, such as, for example, any form described in the present application.

In some embodiments, to secure the tip protector 100 to an endoscope, the tip protector is placed in the open position, and an endoscope is placed inside the tip protector, and, after the endoscope is placed inside the tip protector 100, the tip protector is moved to the closed position. The latch member 803 is used to secure the tip protector 100 in the closed position. In some embodiments, the tip protector 100 is manufactured by injection molding, and the injection molded tip protector is removed from the molding in the open position (as shown in FIG. 8). In these embodiments, the tip protector 100 can be secured an endoscope as described above, or the tip protector 100 can be moved to the closed position prior to securing the tip protector to the endoscope, and the endoscope can be inserted in the proximal or distal end of the tip protector and through an opening of the one or more positional membranes. In any of the above-mentioned embodiments, when the tip protector 100 is properly secured to an endoscope, the tip protector 00 is configured such that only the positional membrane contacts the endoscope.

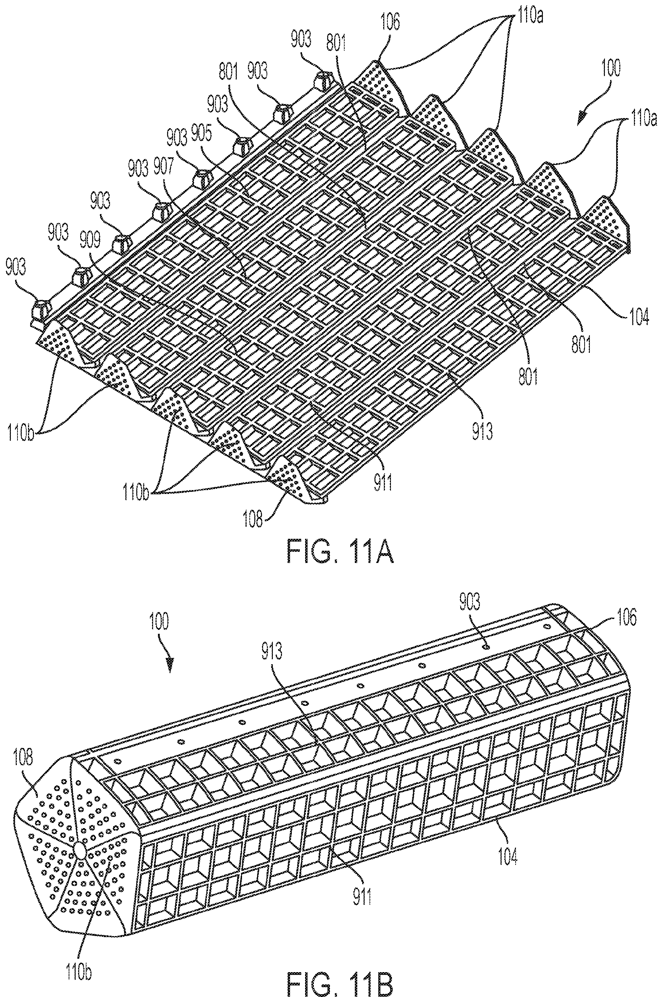

Referring to FIGS. 11A and 11B, the tip protector 100 includes a cage 104 having four hinges 801 and a connecting member 903. The four hinges 801 separate the cage 104 into a first portion 905, a second portion 807, a third portion 909, a fourth portion 911, and a fifth

portion 913. In the illustrated embodiment, the tip protector 100 includes two positional membranes 110a, 110b, in which a portion of each of the membranes 110a 110b is connected to each portion (905, 907, 909, 911, 913). In the illustrated embodiment, a first membrane 110a is positioned at the proximal end 106 of the tip protector 100, and a second membrane 110b is positioned at the distal end 108 of the tip protector. In other embodiments, the tip protector 100 can include only one positional membrane or more than two positional membranes. The positional membranes 110a 110b can take any suitable form, such as, for example, any form described in the present application. The tip protector 100 can be moved between an open position (as shown in FIG. 11A) and a closed position (as shown in FIG. 11B). In the illustrated embodiment, the tip protector 100 is maintained in the closed position by a connecting member 903. The connecting member 903 can be, for example, a clip, friction fit bosses that are configured to be secured to one or more openings of the cage 104, an adhesive, or any other suitable means capable of maintaining the tip protector 100 in the closed position. The connecting member 903 can be made of the same material as the cage 104 or a stiffer material that is configured to better secure the tip protector in the closed position.

In some embodiments, to secure the tip protector 100 to an endoscope, the tip protector is placed in the open position, and an endoscope is placed inside the tip protector. Subsequently, the tip protector is moved to the closed position. In some embodiments, the tip protector 100 is manufactured by injection molding, and the injection molded tip protector is removed from the molding in the open position (as shown in FIG. 11A). In these embodiments, the tip protector 100 can be secured to an endoscope as described above, or the tip protector 100 can be moved to the closed position prior to securing the tip protector to the endoscope, and the endoscope can be secured to the tip protector by inserting it in the proximal or distal end of the tip protector and through an opening of the one or more positional membranes 110a, 110b. In some embodiments, the tip protector 100 is manufactured using flexible sheet stock and/or a flexible tube, in which the flexible sheet stock and/or flexible tube are cut to length with holes and flaps (or other connecting features) punched thereon. In any of the above-mentioned embodiments, when the tip protector 100 is properly secured to an endoscope, the tip protector 100 is configured such that the only contact points between the endoscope and the tip protector are by the positional membrane 110.

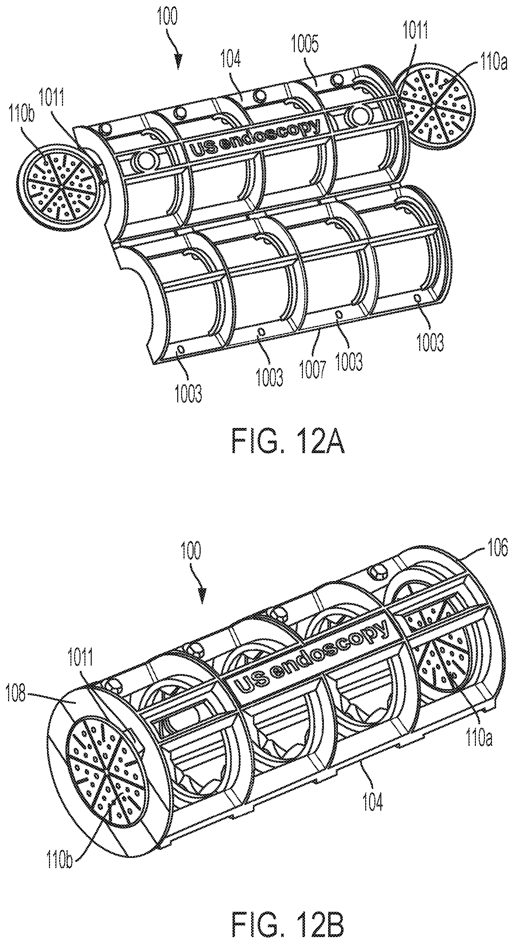

Referring to FIGS. 12A and 12B, the tip protector 100 includes a cage 104 having a hinge 1001 and a connecting member 1003. The hinge 801 separates the tip protector into a first portion 1005 and a second portion 1007. The tip protector 100 includes two positional membranes 110a, 110b in which each of the membranes 110 is connected to the first portion 805 of the cage by a hinge 1011. The first membrane 110a is positioned at the proximal end 106 of the tip protector 100, and the second membrane 110b is positioned at the distal end 108 of the tip protector. In other embodiments, the tip protector 100 can include only one positional membrane or more than two positional membranes. The positional membranes 110a, 110b can take any suitable form, such as, for example, any form described in the present application. In the illustrated embodiment, the connecting member 1003 is joined to the first portion 1005 by friction fit standing bosses that engage holes of the cage 104. The connecting member 1003, however, can be joined to the first portion 1005 by any suitable means, such as, for example, gluing, ultrasonic welding, or any other suitable connection means capable of achieving closure.

In some embodiments, the tip protector 100 is manufactured by injection molding, and the injection molded tip protector is removed from the molding in the open position (as shown in FIG. 12A). The tip protector 100 can be secured to an endoscope by inserting the endoscope in the proximal or distal end of the tip protector and through an opening of the one or more positional membranes 110a, 110b such that the endoscope is within a channel defined by the cage 104. When the tip protector 100 is properly secured to an endoscope, the tip protector is configured such that the only contact points between the endoscope and the tip protector are by the one or more positional membranes 110 of the tip protector.

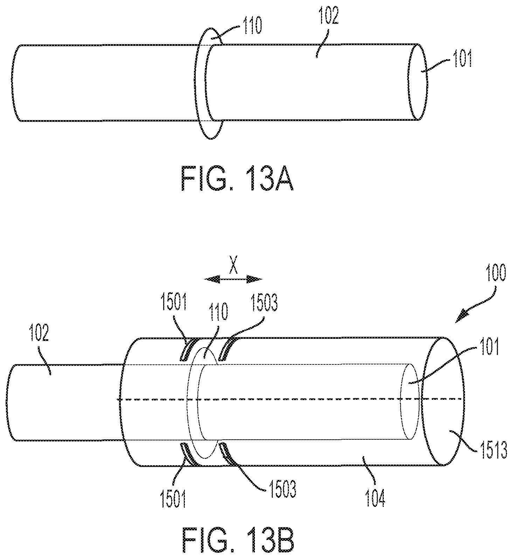

While the various embodiments disclosed herein describe a tip protector having a positional membrane being integrally molded or bonded to the cage of a tip protector membrane it should be understood that the tip protector can include a free-standing positional membrane that is secured directly to an endoscope and a cage that engages the free-standing positional membrane such that the cage is positioned to protect the distal tip of the endoscope. For example, referring to FIGS. 13A and 13B, a tip protector 100 can include a cage 104 and at least one positional membrane 110. The positional membrane 110 can take any suitable form that is capable of being attached to an endoscope 102, such as, for example, any form described in the present application. The cage 104 can take any suitable form that is capable of protecting the distal tip of an endoscope and capable of allowing air flow to facilitate drying of any residual moisture on the attached endoscope 102. For example, the cage 104 can take any form described in the present application. The free-standing positional membrane 110 can attach to the endoscope 102 by any suitable means, such as, for example any means described in the present application, or in some embodiments, by a friction fit and/or pocket provided in the outer cage..

In one exemplary embodiment, the cage 104 and the positional membrane 110 are configured such that there is a friction fit between the positional membrane 110 and the inner wall 1513 of the cage. In some embodiments, such as the illustrated embodiment, the cage 104 includes a first stop member 1501 and a second stop member 1503, in which each of the first and second stop members 1501, 1503 engage the positional membrane 110 to maintain the cage 104 in a desired position. The stop members 1501, 1503 can be, for example, one or more protrusions that extend inward from an inner wall 1513 of the cage 104. The dimensions of the positional membrane 110 (e.g., the width, outer diameter, etc.) is smaller than the dimensions of the inner surface 1513 of the cage 104 (e.g., width, diameter of inner surface 1513, etc) such that the positional membrane 110 can move axially within the cage 104. The stop members 1501, 1503 extend inward from the inner wall 1513 of the cage 104 such that the stop members engage the positional membrane 110 to prevent the positional membrane from moving within the cage. In the illustrated embodiment, the cage 104 is placed in engagement with the positional membrane 110 such that the positional membrane is disposed between the first stop member 1501 and the second stop member 1503. In this embodiment, when the cage 104 is engaged with the positional membrane 110 the cage 104 can move axially in the direction X, but the stop members 1501, 1503 maintain the cage 104 in a desired position that protects the endoscope 102 and prevents the cage 104 from disengaging the positional membrane 110.

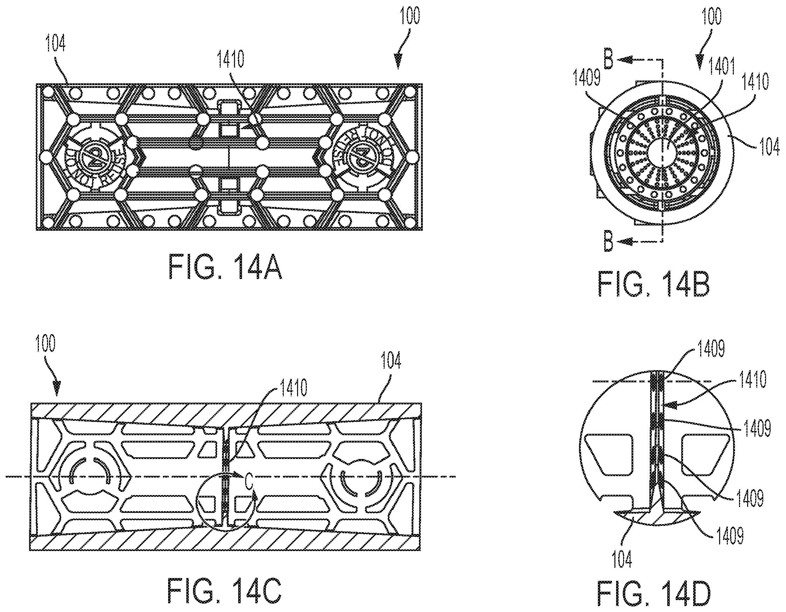

Referring to FIGS. 14A-14C, another embodiment of a tip protector 100 includes a cage 104 and one or more annular diaphragms 1410 (instead of the positional membranes described herein). The tip protector 100 can include the features of any of the tip protectors described in the present application. The annular diaphragm 1410 can be connected, joined, or integral to the cage 104 and is configured to connect the tip protector 100 to an endoscope by a frictional fit. That is, the annular diaphragm 1410 has a main opening 1401 that is sized to provide a frictional fit to an endoscope. Similar to the positional membranes described herein, the annular diaphragm 1410 may include a plurality of air flow openings 1409 that allow for air flow through the annular diaphragm such that any residual moisture can dry or exit during storage of the endoscope. Also similar to the positional membranes described herein, the annular diaphragm 1410 can be configured such that the only contact between an endoscope and the tip protector 100 is between the annular diaphragm 1410 and the endoscope, and the tip protector 100 can be configured such that the contact point between the annular diaphragm 1410 and the endoscope is away from the exit of the accessory channel at the scope's distal end.

While the tip protector 100 having an annular diaphragm 1410 is contrary to the positional membrane design that is targeted to allow a single tip protector to be used across a variety of scope diameters, the annular diaphragm 1410 design may use color coding and/or other visual cues that allow a user to quickly determine the inventory of clean, ready to use endoscopes. The annular diaphragm 1410 design could also allow the fabrication of a rigid cage by employing a thermoplastic elastomer or silicone that is glass-filled, in which the annular diaphragm 1410 includes very small features that do not allow glass fibers to enter. This will allow the annular diaphragm 1410 to be resin-rich and soft (due to the absence of glass fibers), in contrast to the cage 104 that is substantially more rigid.

The various embodiments described in the present application describe a tip protector 100 having a cage 104 and either a positional membrane 110 or an annular diaphragm 1410, and the various embodiments described herein include different ways in which the positional membrane 110 or annular diaphragm 1410 engages or connects with the cage 104 such that the cage 104 is positioned to protect a distal tip of an endoscope when the positional membrane or annular diaphragm secures the tip protector to the endoscope. For example, the positional membrane 110 or annular diaphragm 1410 can be integrally molded with the cage 104, bonded (e.g., by over-molding, ultrasonic welding, mechanically locking, etc.) to the cage 104, connected to the cage 104 by a friction fit, free-standing and engage one or more stop members of the cage 104 to maintain the cage in a desired position, or can engage with the cage 104 by any other suitable means that allows the cage to be positioned to protect the distal tip of an endoscope. When the positional membrane 110 or annular diaphragm 1410 is described as being "engaged with the cage," "attached to the cage," "connected to the cage," or any other form of being contacted with the cage 104, it should be understood that this description includes any of the above-mentioned embodiments of the connection between positional membrane 110 or annular diaphragm 1410 and the cage 104 that allows the cage 104 to be positioned to protect an endoscope.

The surface of any of the tip protectors 100 described in the present application can be treated with antimicrobial agents to better maintain a claim, ready-to-use state, to prevent the tip protectors from introducing contaminants to an endoscope, and/or to prevent bacteria from growing on an endoscope. The antimicrobial agent can be silver ions, copper salts, zinc pyrithione, and other organics like phenolic biocides. It should be noted that nano-sized texture and/or surface geometry can be bred to inhibit bacteria growth. In certain embodiments, any of the tip protectors described herein can be treated with a hydrophobic coating or hydrophobic additives that are configured to encourage moisture to migrate to an area where it can evaporate. In some embodiments, the tip protectors 100 described herein can include a color- changing additive that acts as a cleanliness monitor for the tip protector.

The color-changing additives can sense moisture and/or react with bacteria such that a portion of the tip protector changes colors if the tip protector or the endoscope on which the tip protector is connected has been compromised due to exposure, growth, inadequate disinfection, or the like. In these embodiments, if bacteria grow at the contact point(s) between the tip protector and the endoscope, the color changing additives will indicate the bacteria growth. The color-changing additive could also include a chromogenic or conjugated electrochromic configured to react to environmental changes at the interface of the tip protector and endoscope.

While all the tip protectors 100 described herein include different embodiments for the cage 104 and the positional membrane(s) 110 or annular diaphragm(s) 1410, it should be understood that the features of each of these embodiments can be used with any of the embodiments of the tip protector. In addition, while the tip protectors 100 of the present application are described as being used to protect a distal tip of an endoscope, it should be understood that the tip protectors can be used to protect various other types of medical devices, such as, for example, a duodenoscope (FIG. 14), an ultrasonic endoscope, a bronchoscope, a rigid ureteroscope, or any other suitable medical device that requires protection during transportation and storage.

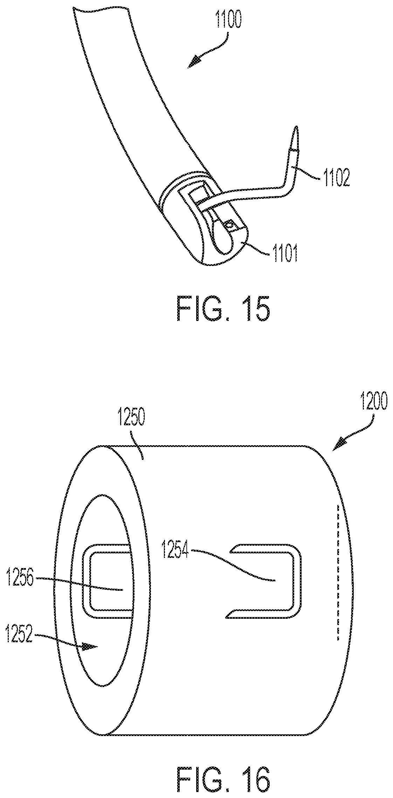

FIGS. 15 and 16 illustrate a cap 1200 for protecting an elevator 1102 of a duodenoscope 1100. Referring to FIG. 15, a distal tip 1101 of a duodenoscope includes various components, including a camera (which is connected to the elevator 1102). The angle of the elevator 1102 relative to the distal tip 1101 of the duodenoscope 1100 can be adjusted to provide various angles for changing the attack angle of the devices used through the accessory channel. During storage of the duodenoscope 1100, the elevator is at least partially retracted into the distal tip 1101 (e.g., a position in line with the scope axis of the distal tip 1101). As a result, residual moisture can become trapped between the elevator 1102 and the distal tip 1101, which can cause bacteria growth. In addition, because this area is hard to reach with commonly available cleaning tools, contamination can reside in these regions, threatening subsequent patients.

Referring to FIG. 16, an exemplary embodiment of cap 1200 is placed on the elevator 1102 of the duodenoscope (during storage) to prevent bacteria growth between the elevator and the distal tip 1101 due to residual moisture. The cap 1200 includes a body member 1250 defining a channel 1252, a first tab 1254, and a second tab 1256. The cap 1200 is placed on the elevator 1102 such that the tabs 1254, 1256 contact the elevator 1102. The tabs 1254, 1256 can be treated with an antimicrobial to prevent bacteria from growing between the elevator 1102 and the distal tip 1101 of the duodenoscope 1100. The antimicrobial can be, for example, silver ions, copper salts, zinc pyrithione, and other organics like phenolic biocides

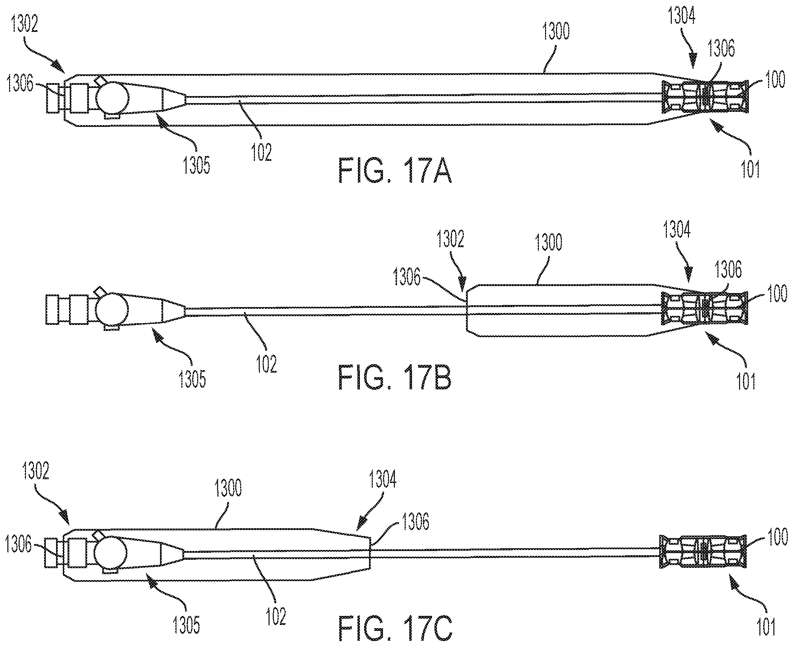

FIGS.17-17C illustrate various exemplary embodiments of a protective sleeve 1300 for protecting an endoscope 102 during transportation and storage. The protective sleeve 1300 is configured to isolate the exterior of the endoscope from dust, dirt, debris, moisture, or any other substance that can contaminate the endoscope. In certain embodiments, the protective sleeve 1300 includes a proximal end 1302, a distal end 1304, and at least one attaching member 1306 that is configured to secure the protective sleeve 1300 to the endoscope 102. The attaching member 1306 can be, for example, an elastic band, a drawstring, hook, or clamp, etc. In the illustrated embodiments, the protective sleeve 1300 includes an attaching member 1306 at the

proximal end 1302 and the distal end 1304 of the protective sleeve, which allows both the proximal end 1302 and the distal end 1304 of the protective sleeve to be secured to the endoscope 102. In some embodiments, only one of the proximal end 1302 and the distal end 1304 of the protective sleeve 1300 include an attaching member 1306. In some embodiments, the protective sleeve 1300 can include an attaching member 1306 at a portion of the protective sleeve 1300 that is between the proximal end 1302 and the distal end 1304 The protective sleeve 1300 can be a plastic film sleeve. The protective sleeve 1300 can be made from, for example, polyethylene, polyurethane, polyester, woven open-cell fabric, cellulose, etc.

Referring to FIG. 17A, in certain embodiments, the protective sleeve 1300 is attached to the endoscope 102 such that the protective sleeve extends along the entire endoscope. That is, the protective sleeve 1300 protects the control head 1305 at the proximal end of the endoscope 102, and the distal tip 101 at the distal end of the endoscope 102. Referring to FIG. 17B, in some embodiments, the protective sleeve 1300 is attached only to a distal end of the endoscope 102 such that the protective sleeve protects the distal tip 101 of the endoscope. In the above-mentioned embodiments, the protective sleeve 1300 can be configured to attach to a tip protector 100 at the distal end of the endoscope 102. Referring to FIG. 17C, in certain embodiments, the protective sleeve 1300 is attached only to a proximal end of the endoscope 102 such that the protective sleeve protects the control head 1305. In some embodiments, multiple protective sleeves 1300 can be used to protect an endoscope 102. That is, referring to FIGS. 17B and 17C one protective sleeve (as shown in FIG. 17B) can be used to protect the proximal end and another protective sleeve (as shown in FIG. 17C) can be used to protect the distal end.

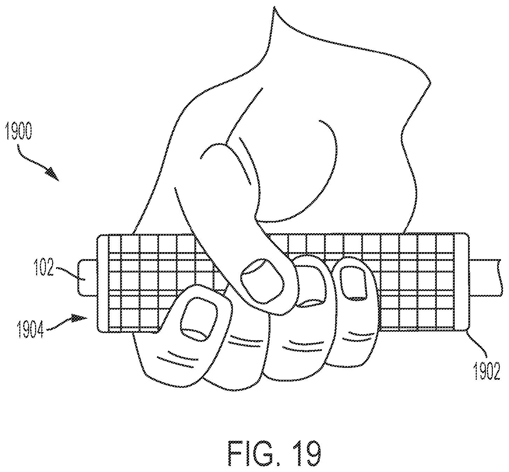

With continued reference to the figures, and now with reference to FIG. 19, in some embodiments, the tip protector 100 may be a gripping and/or actuation device or apparatus 1900 configured to enhance or otherwise achieve a more efficient and ergonomic gripping and actuation of an endoscope (or similar medical instrument) during a medical procedure (e.g., an endoscopy procedure). The gripping device 1900 include a body 1902 having a proximal end and distal end. The body 1902 may be tubular (e.g., have a tube-like shape or structure) and be formed from materials known in the art to achieve a flexible geometry of the gripping device 1900. It should be appreciated that additional shapes (e.g., polygonal) for the body 1902 may be provided, for example, based on the use of the gripping device 1900 and/or the configuration of the medical instrument.

In some embodiments, the body 1902 may be similar to the cage 104 in that the body 1902 may include a hollow core or similar channel 1904 defined in the body 1902 and extending between the proximal end and the distal end. The body 1902 may further include one or more openings, for example, at the proximal and/or distal end of the channel for at least partially receiving the endoscope therein (e.g., within the channel 1904 of the body 1902). In some embodiments, a first opening to the channel 1904 may be provided at the proximal end of the body 1902, and more particularly, the channel 1904. It should be appreciated that the first opening and/or the channel 1904 may be sized or otherwise shaped for at least partially receiving the endoscope therebetween.

Additionally, or alternatively, the gripping device 1900 may further include a positional membrane 110 adapted or otherwise sized to be disposed within the channel 1904 (hollow core) of the body 1902. The positional membrane 110 may include an opening at the proximal end for at least partially receiving the endoscope therebetween. It should be appreciated that the opening may be sized or otherwise shaped for receiving the endoscope (or similar medical instrument) therebetween. In some embodiments, the opening of the positional membrane 110 may be smaller than the opening of the channel 1904 for frictionally engaging the endoscope when inserting the endoscope within the opening of the positional membrane 110.

One or more of the components of the gripping device 1900, for example, the body 1902 and/or positional member 110, may be formed from the same or similar materials for maintain the flexible geometry of the gripping device 1900. Additionally, or alternatively, different materials may be used for forming the components as long as the materials forming the components allow for the gripping device 1900 to maintain the flexible geometry. It should be appreciated that the flexible geometry of the body 1902 or the gripping and/or actuation device 1900 may allow for operators of the endoscope to achieve a more efficient and ergonomic gripping and actuation of the endoscope during a procedure.

While various inventive aspects, concepts and features of the inventions may be described and illustrated herein as embodied in combination with exemplary embodiments, these various aspects, concepts and features may be used in many alternative embodiments, either individually or in various combinations and subcombinations thereof. Unless expressly excluded herein, all such combinations and subcombinations are intended to be within the scope of the present inventions.

## Claims

1. A tip protector (100) for removably attaching to a distal tip (101) of an endoscope, the tip protector comprising:
a cage (104) having a proximal end (106), a distal end (108), and a plurality of holes (107) disposed between the proximal end and the distal end, wherein the cage defines a channel (112) that extends from the proximal end to the distal end; and
a positional membrane (110) connected to the cage such that a portion of the channel (112) extends between the positional membrane (110) and the distal end (108) of the cage (104), wherein the positional membrane comprises a main opening (111) for receiving the distal tip of the endoscope, a plurality of web members (215) that surround the main opening, and a plurality of air flow openings (109) included in the plurality of web members (215) for allowing air flow through the positional membrane, wherein each web member of the plurality of web members (215) is separated from an adjacent web member by a slot (217);
wherein the positional membrane (110) is configured to removably attach the tip protector (100) to the endoscope such that the distal tip (101) of the endoscope extends beyond the positional membrane (110) and is disposed within the portion of the channel (112) that extends between the positional membrane (110) and the distal end (108) of the cage (104).

2. The tip protector of claim 1, wherein the distal end (108) of the cage (104) is open.

3. The tip protector of claim 1, wherein the tip protector (100) is configured such that the endoscope can be inserted in either the proximal end (106) or the distal end (108) of the tip protector to secure the tip protector (100) to the endoscope.

4. The tip protector of claim 1, wherein the positional membrane (110) is made from a flexible material, such as thermoplastic elastomer.

5. The tip protector of claim 1, wherein the tip protector (100) is a single, injection-molded piece.

6. The tip protector of claim 1, wherein the tip protector (100) includes one or more hinges (801) that allow the tip protector to move between an open position and a closed position.

7. The tip protector of claim 1, wherein at least one of the cage (104) and the positional membrane (110) is treated with an antimicrobial.

8. The tip protector of claim 1, wherein the positional membrane (110) is attached to the cage (104) at a center point along a length of the cage (104).

9. The tip protector of claim 1, wherein the positional membrane (110) is bonded to the cage (104).

10. The tip protector of claim 1, wherein the positional membrane (110) is free-standing.

11. The tip protector of claim 10, wherein, the cage (104) comprises one or more stop members (1501, 1503) configured to engage the positional membrane (110) to maintain the cage (104) in a desired position when the tip protector (100) is secured to the endoscope.

12. The tip protector of claim 1, wherein at least a portion of the tip protector is treated with a hydrophobic material.

13. The tip protector of claim 1, wherein the cage (104) is rigid.

14. The tip protector of claim 1, wherein the positional membrane (110) comprises an annular diaphragm (1410).

## Patentansprüche

1. Spitzenschutz (100) zum entfernbaren Anbringen an einer distalen Spitze (101) eines Endoskops, wobei der Spitzenschutz umfasst:
einen Käfig (104) mit einem proximalen Ende (106), einem distalen Ende (108) und einer Vielzahl von Löchern (107), die zwischen dem proximalen Ende und dem distalen Ende angeordnet ist, wobei der Käfig einen Kanal (112) definiert, der sich von dem proximalen Ende zu dem distalen Ende erstreckt; und
eine Positionsmembran (110), die mit dem Käfig derart verbunden ist, dass sich ein Teil des Kanals (112) zwischen der Positionsmembran (110) und dem distalen Ende (108) des Käfigs (104) erstreckt, wobei die Positionsmembran eine Hauptöffnung (111) zum Aufnehmen der distalen Spitze des Endoskops, eine Vielzahl von Stegelementen (215), die die Hauptöffnung umgeben, und eine Vielzahl von Luftstromöffnungen (109), die in der Vielzahl von Stegelementen (215) beinhaltet ist, um einen Luftstrom durch die Positionsmembran zu ermöglichen, umfasst, wobei jedes Stegelement der Vielzahl von Stegelementen (215) von einem benachbarten Stegelement durch einen Schlitz (217) getrennt ist;
wobei die Positionsmembran (110) konfiguriert ist, um den Spitzenschutz (100) entfernbar an dem Endoskop anzubringen, sodass sich die distale Spitze (101) des Endoskops über die Positionsmembran (110) hinaus erstreckt und innerhalb des Teils des Kanals (112) angeordnet ist, der sich zwischen der Positionsmembran (110) und dem distalen Ende (108) des Käfigs (104) erstreckt.

2. Spitzenschutz nach Anspruch 1, wobei das distale Ende (108) des Käfigs (104) offen ist.

3. Spitzenschutz nach Anspruch 1, wobei der Spitzenschutz (100) derart konfiguriert ist, dass das Endoskop entweder in das proximale Ende (106) oder das distale Ende (108) des Spitzenschutzes eingeführt werden kann, um den Spitzenschutz (100) an dem Endoskop zu sichern.

4. Spitzenschutz nach Anspruch 1, wobei die Positionsmembran (110) aus einem flexiblen Material, wie etwa thermoplastischem Elastomer, hergestellt ist.

5. Spitzenschutz nach Anspruch 1, wobei der Spitzenschutz (100) ein einzelnes, spritzgegossenes Stück ist.

6. Spitzenschutz nach Anspruch 1, wobei der Spitzenschutz (100) ein oder mehrere Scharniere (801) beinhaltet, die es dem Spitzenschutz ermöglichen, sich zwischen einer offenen Position und einer geschlossenen Position zu bewegen.

7. Spitzenschutz nach Anspruch 1, wobei wenigstens eines von dem Käfig (104) und der Positionsmembran (110) mit einem antimikrobiellen Mittel behandelt ist.

8. Spitzenschutz nach Anspruch 1, wobei die Positionsmembran (110) an dem Käfig (104) an einem Mittelpunkt entlang einer Länge des Käfigs (104) angebracht ist.

9. Spitzenschutz nach Anspruch 1, wobei die Positionsmembran (110) an den Käfig (104) gebunden ist.

10. Spitzenschutz nach Anspruch 1, wobei die Positionsmembran (110) freistehend ist.

11. Spitzenschutz nach Anspruch 10, wobei der Käfig (104) ein oder mehrere Anschlagelemente (1501, 1503) umfasst, die konfiguriert sind, um mit der Positionsmembran (110) in Eingriff zu kommen, um den Käfig (104) in einer gewünschten Position zu halten, wenn der Spitzenschutz (100) an dem Endoskop gesichert ist.

12. Spitzenschutz nach Anspruch 1, wobei wenigstens ein Teil des Spitzenschutzes mit einem hydrophoben Material behandelt ist.

13. Spitzenschutz nach Anspruch 1, wobei der Käfig (104) starr ist.

14. Spitzenschutz nach Anspruch 1, wobei die Positionsmembran (110) ein ringförmiges Diaphragma (1410) umfasst.

## Revendications

1. Protecteur de pointe (100) destiné à être fixé de manière amovible à une pointe distale (101) d'un endoscope, le protecteur de pointe comprenant :
une cage (104) ayant une extrémité proximale (106), une extrémité distale (108) et une pluralité de trous (107) disposés entre l'extrémité proximale et l'extrémité distale, dans lequel la cage délimite un canal (112) qui s'étend de l'extrémité proximale à l'extrémité distale ; et
une membrane positionnelle (110) reliée à la cage de telle sorte qu'une partie du canal (112) s'étende entre la membrane positionnelle (110) et l'extrémité distale (108) de la cage (104), dans lequel la membrane positionnelle comprend une ouverture principale (111) destinée à recevoir la pointe distale de l'endoscope, une pluralité de nervures (215) qui entourent l'ouverture principale et une pluralité d'ouvertures de circulation d'air (109) incluses dans la pluralité de nervures (215) pour permettre la circulation d'air à travers la membrane positionnelle, dans lequel chaque nervure de la pluralité de nervures (215) est séparée d'une nervure adjacente par une fente (217) ;
dans lequel la membrane positionnelle (110) est configurée pour fixer de manière amovible le protecteur de pointe (100) à l'endoscope de sorte que la pointe distale (101) de l'endoscope s'étende au-delà de la membrane positionnelle (110) et soit disposée à l'intérieur de la partie du canal (112) qui s'étend entre la membrane positionnelle (110) et l'extrémité distale (108) de la cage (104).

2. Protecteur de pointe selon la revendication 1, dans lequel l'extrémité distale (108) de la cage (104) est ouverte.

3. Protecteur de pointe selon la revendication 1, dans lequel le protecteur de pointe (100) est configuré de telle sorte que l'endoscope puisse être inséré soit dans l'extrémité proximale (106), soit dans l'extrémité distale (108) du protecteur de pointe pour fixer le protecteur de pointe (100) à l'endoscope.

4. Protecteur de pointe selon la revendication 1, dans lequel la membrane positionnelle (110) est constituée d'un matériau flexible, tel qu'un élastomère thermoplastique.

5. Protecteur de pointe selon la revendication 1, dans lequel le protecteur de pointe (100) est une pièce unique moulée par injection.

6. Protecteur de pointe selon la revendication 1, dans lequel le protecteur de pointe (100) comporte une ou plusieurs charnières (801) qui permettent au protecteur de pointe de se déplacer entre une position ouverte et une position fermée.

7. Protecteur de pointe selon la revendication 1, dans lequel au moins l'une de la cage (104) et de la membrane positionnelle (110) est traitée avec un agent antimicrobien.

8. Protecteur de pointe selon la revendication 1, dans lequel la membrane positionnelle (110) est fixée à la cage (104) au niveau d'un point central dans le sens d'une longueur de la cage (104).

9. Protecteur de pointe selon la revendication 1, dans lequel la membrane positionnelle (110) est liée à la cage (104).

10. Protecteur de pointe selon la revendication 1, dans lequel la membrane positionnelle (110) est autoportante.

11. Protecteur de pointe selon la revendication 10, dans lequel la cage (104) comprend un ou plusieurs éléments d'arrêt (1501, 1503) configurés pour venir en prise avec la membrane positionnelle (110) pour maintenir la cage (104) dans une position souhaitée lorsque le protecteur de pointe (100) est fixé à l'endoscope.

12. Protecteur de pointe selon la revendication 1, dans lequel au moins une partie du protecteur de pointe est traitée avec un matériau hydrophobe.

13. Protecteur de pointe selon la revendication 1, dans lequel la cage (104) est rigide.

14. Protecteur de pointe selon la revendication 1, dans lequel la membrane positionnelle (110) comprend un diaphragme annulaire (1410).
